(19) Europäisches Patentamt
European Patent Office
Office européen des brevets

(11) Veröffentlichungsnummer: **0 207 097**
**B1**

(12) **EUROPÄISCHE PATENTSCHRIFT**

(45) Veröffentlichungstag der Patentschrift: **08.08.90**

(51) Int. Cl.$^5$: **C 07 C 233/01**, A 61 K 31/27, A 61 K 31/16, A 61 K 31/265

(21) Anmeldenummer: **85906030.3**

(22) Anmeldetag: **13.12.85**

(86) Internationale Anmeldenummer:
**PCT/CH85/00175**

(87) Internationale Veröffentlichungsnummer:
**WO 86/03747 03.07.86 Gazette 86/14**

(54) **NEUE KOHLENSÄUREESTER.**

(30) Priorität: **19.12.84 CH 6015/84**
**01.02.85 CH 454/85**

(43) Veröffentlichungstag der Anmeldung:
**07.01.87 Patentblatt 87/02**

(45) Bekanntmachung des Hinweises auf die Patenterteilung:
**08.08.90 Patentblatt 90/32**

(84) Benannte Vertragsstaaten:
**AT BE CH DE FR GB IT LI LU NL SE**

(56) Entgegenhaltungen:
**US-A-3 634 407**

(73) Patentinhaber: **CIBA-GEIGY AG**
**Klybeckstrasse 141**
**CH-4002 Basel (CH)**

(72) Erfinder: **PETER, Heinrich**
**Bündtenweg 69**
**CH-4102 Binningen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft neue, durch Kohlensäuremonoester acylierte Derivate von Hydroxamsäuren, insbesondere von Trihydroxamsäuren, welche unter der Bezeichnung Ferrioxamine und speziell Desferrioxamin Stoffwechselprodukte von Mikroorganismen, insbesondere Actinomyceten, bekannt sind, und darunter vor allem von Kohlensäuremonoestern abgeleitete N- und/oder O-Acylate von Desferrioxamin B der allgemeinen Formel

```
                  O-AA1                          O-AA2                          O-AA3
                  |                              |                              |
B-NH-(CH2)5-N-C-CH2CH2-C-NH-(CH2)5-N-C-CH2CH2-C-NH-(CH2)5-N-C-CH3
                    ||        ||                   ||        ||                   ||
                    O         O                    O         O                    O
```

(I)

mit der folgenden Bedeutung der Symbole:

$AA^1$, $AA^2$ und $AA^3$, jedes unabhängig, ist ein als Ac bezeichneter Acylrest einer Carbonsäure mit 1—24 C-Atomen oder ein als Cb bezeichneter veresterter Oxycarbonylrest (Acylrest eines Kohlensäuremonoesters) mit insgesamt 2—25 C-Atomen, und

B hat eine von Ethoxycarbonyl und Benzyloxycarbonyl unterschiedliche Bedeutung von Cb oder, falls mindestens eines der Symbole $AA^1$, $AA^2$ und $AA^3$ für Cb steht, kann auch Wasserstoff oder eine Aminoschutzgruppe, ausgewählt aus mono- oder bicyclischen α-Arylniederalkoxycarbonylresten, die von einem Benzyloxycarbonylrest verschieden sind, aus sekundären oder tertiären Alkoxycarbonyl- oder Cycloalkoxycarbonylresten, sowie aus β-(Tri-hydrocarbyl-silyl)-ethoxycarbonylresten und Allyloxycarbonyl, sein, sowie Salze davon, falls dieses Acylat salzbildende Eigenschaften besitzt.

Die Erfindung betrifft auch Verfahren zur Herstellung der oben genannten Verbindungen sowie pharmazeutische Zusammensetzungen, enthaltend diese Verbindungen und Verfahren zu ihrer Herstellung; ferner auch die Anwendung dieser Verbindungen als chemische Zwischenprodukte, insbesondere zur Herstellung therapeutisch wirksamer Derivate des Desferrioxamins B und die Verwendung dieser Verbindungen zur Herstellung diese enthaltender pharmazeutischer Zusammensetzungen für die Behandlung von krankhaften Zuständen in Warmblütern, einschliesslich Menschen, welche durch Eisen(III)-abhängige pathogene Organismen verursacht sind.

Desferrioxamin B, der Grundstoff der Acylate der vorliegenden Erfindung, ist bereits längere Zeit bekannt (H. Bickel, H. Keberle und E. Vischer: Helv. Chim. Acta *46*, 1385—9 [1963]). Seine chemische Struktur entspricht der Formel

```
                 O-H                            O-H                            O-H   (A)
                 |                              |                              |
NH2-(CH2)5-N-C-CH2CH2-C-NH-(CH2)5-N-C-CH2CH2-C-NH-(CH2)5-N-C-CH3
                   ||        ||                   ||        ||                   ||
                   O         O                    O         O                    O
   1-5         6 7       10 11            17 18       21 22             28 29 30
```

und wird im Einklang mit der Regel C—06 (Austausch-Nomenklatur) der offiziellen IUPAC-Nomenklatur mit dem systematischen Namen 6,17,28-Trihydroxy-7,10,18,21,29-pentaoxo-6,11,17,22,28-pentaazatriacontylamin bezeichnet. (Der Einfachheit halber werden jedoch nachfolgend die Namen der Acylate vom trivialen Namen abgeleitet, wobei die Lage einzelner Acylreste jeweils auf den Aminostickstoff N bzw. die als O, O' und O'' bezeichneten Sauerstoffatome der Hydroxylgruppen in Stellungen 6, 17, bzw. 28 bezogen wird).

In der US—A—3 634 407 werden nur Desferrioxamin B Derivate mit freien OH-Gruppen der Hydroxamsäurereste beansprucht.

Derivate die einen Oxycarbonylrest, z.B. einen Ethoxycarbonyl- oder Benzyloxycarbonylrest, am Sauerstoffatom einer der Hydroxamsäuregruppen tragen, sind in der Patentschrift nicht beschrieben.

Die im Hauptanspruch generisch erwähnten O-unsubstituierten N-Benzyloxycarbonyl- und N-Ethoxycarbonylderivate werden mit den anderen im Anspruch generisch erwähnten Verbindungen gleichgestellt. In der Patentschrift wird nicht gesagt, dass sich die letztgenannten Verbindungen durch irgendwelche vorteilhaften pharmakologischen Eigenschaften von Desferrioxamin B unterscheiden. In Spalte 21, Zeilen 21—26, sowie in Spalte 20, Zeile 51, der US—A—3 634 407 wird nur die intravenöse und intramuskuläre, nicht aber die orale Applikation erwähnt. Ein Vergleich der in der letzten Zeile von Spalte 20 gegebenen lethalen Dosen von Desferrioxamin B bei intravenöser Verabreichung ($LD_{50}$ = 340 mg/kg) mit dem in Spalte 21, Zeilen 1 und 2, angeführten Befund, dass bei oraler Verabreichung von 3 g/kg keinerlei toxische Erscheinungen auftreten, legt die Vermutung nahe, dass Desferrioxamin B oral gar nicht resorbiert wird und daher auch nicht toxisch sein kann.

Zu den markantesten Eigenschaften von Desferrioxamin B und seinen Additionssalzen, die mit einem Aequivalent Säure gebildet werden, gehört die Fähigkeit, sich zu stabilen Chelat-artigen Metallkomplexen insbesondere mit trivalenten Metallionen, wie Chrom(III)-Aluminium- und in erster Linie Eisen(III)-Ionen, zu verbinden. Dies verleiht Desferrioxamin B die wertvolle pharmakologische Wirksamkeit, die Ablagerung eisenhaltiger Pigmente im Gewebe zu verhindern und bei bestehenden Eisenablagerungen im Organismus

eine Ausscheidung des Eisens zu bewirken, z.B. bei Hämochromatose, Hämosiderose, Lebercirrhose und Vergiftungen mit Verbindungen des dreiwertigen Eisens. Die breite therapeutische Anwendung von Desferrioxamin B und seinen Salzen (z.B. insbesondere vom Methansulfonat) erstreckt sich deshalb allgemein auf Krankheiten und krankhafte Zustände des menschlichen Körpers (sowie des Körpers anderer Warmblüter), welche mit übermässiger Belastung des Organismus mit Eisen(III)-Ionen ($Fe^{+++}$-Ionen) einhergehen, wie Thalassämie major, Sichelzell-Anämie, sideroachrestische Anämie, aplastische Anämie und weitere anämische Formen, in welche Hämosiderose (d.h. eine lokale oder allgemeine, Erhöhung der Eisenvorräte in sonst unbeschädigten Körpergeweben) eine Rolle spielt. Zu diesem Typus gehören auch krankhafte Zustände, die sich in Patienten nach mehrmaligen Bluttransfusionen oder mehrfach wiederholter Dialyse-Behandlung bei fehlender oder geschädigter Nierenfunktion entwickeln. Dank den komplexbildenden Eigenschaften erwies Desferrioxamin B eine bedeutende Wirksamkeit bei Erkrankungen durch Eisen(III)-abhängige Mikroorganismen und Parasiten, wie insbesondere bei Malaria, die nicht nur in humaner Medizin, sondern auch in Tiermedizin von prinzipieller Wichtigkeit ist. Auch die Komplex-Bildung mit anderen dreiwertigen Metallen kann zu deren Ausscheidung aus dem Organismus verwendet werden, z.B. zur Entfernung von Aluminium bei der Dialyse-Encephalopathie und Osteomalacia, sowie bei der Alzheimer Krankheit.

Als ein schwerwiegender Nachteil erweist sich jedoch die Tatsache, dass Desferrioxamin und seine Salze bei oraler Gabe nur eine geringe, unzureichende Wirksamkeit aufweisen und bei allen oben genannten Anwendungsmöglichkeiten eine parenterale Verabreichungsform benötigen. So ist z.B. als eine besonders wirksame Methode empfohlen, die Wirksubstanz mittels einer langsamen (8- bis 12-stündigen) subkutanen Infusion zu verabreichen, was aber entweder Hospitalisierung des Patienten oder, bei ambulanter Behandlung, die Anwendung einer tragbaren mechanischen Vorrichtung, wie einer durch elektrischen Antrieb betätigten Infusionsspritze, bedingt. Abgesehen von ihrer Umständlichkeit sind solche Lösungen mit hohen Behandlungskosten behaftet, was ihre Anwendung stark einschränkt, insbesondere wird eine umfassende Behandlung der Thalassämie in den Ländern des Mittelmeerraums, des Mittleren Ostens, Indiens und Südostasiens, der Malaria weltweit und der Sichelzell-Anämie in den afrikanischen Ländern verunmöglicht. Diese weit verbreiteten Krankheiten stellen weiterhin ein schwerwiegendes problem für das Gesundheitswesen in dies Ländern dar und machen die Suche nach einer einfacheren und billigeren Therapie, vorzugsweise mittels eines oral wirksamen Präparats, zur vordringlichen Aufgabe auf diesem Gebiet.

Bisher wurden jedoch keine oral wirksamen Darreichungsformen von Desferrioxamin B beschrieben und für seine bisher bekannten Derivate, wie N,O,O′,O″-Tetraacylate und N-Monoacylate, sind weder in der oben erwähnten Mitteilung von Bickel et al. noch anderswo einschlägige Angaben über ihre biologische Wirksamkeit zu finden. Auf Grund theoretischer Vorstellungen ist anzunehmen, dass zur Chelatisierung von Metallionen und somit zu therapeutisch anwendbaren Metallkomplex-Bildung die freien Hydroxylgruppen des Desferrioxamins B den wesentlichen strukturellen Beitrag leisten. Wenn man sie aber durch Acylierung blockiert und somit ihrer Teilnahme an Komplexbildung praktisch entzieht, ist es zu erwarten, dass solche O,O′,O″-Triacylate und ähnliche Verbindungen mit blockierten Hydroxylgruppen, wenn überhaupt, dann nur in einem sehr geringen Masse komplexbildende Eigenschaften und demzufolge auch die wesentliche Voraussetzung für die therapeutische Anwendung besitzen können.

Im Gegensatz zu diesen Ueberlegungen wurde nun gefunden, dass in denselben Indikationen, in welchen bisher Desferrioxamin B, z.B. in Form des eingeführten Handelspräparats Desferal®, nur mittels parenteraler Darreichungsformen wirksam war, die oben charakterisierten, durch Kohlensäuremonoester (veresterte Oxycarbonylreste) acylierten Derivate der Formel I analoge Wirkungen überraschenderweise bei oraler Verabreichung aufweisen, d.h. unter Bedingungen, die bisher als unerreichbar günstig gegolten haben.

Gegenstand der vorliegenden Erfindung sind in erster Linie die von Kohlensäuremonoestern abgeleiteten Acylate der eingangs definierten Formel I (einschliesslich entsprechender Salze), insbesondere diejenigen mit den folgenden Bedeutungen der Symbole in Formel I: $AA^1$, $AA^2$ und $AA^3$, jeweils alle zusammen, sind Acylreste, welche, jeder unabhängig von den anderen, eine Bedeutung von Ac haben, oder aber Oxycarbonylreste, welche jeder unabhängig von anderen, eine Bedeutung von Cb haben. Darunter sind bevorzugt Verbindungen der Formel I, worin $AA^1$, $AA^2$ und $AA^3$ identisch sind und jeweils alle für dasselbe Ac oder dasselbe Cb stehen.

Der von einer Carbonsäure abgeleitete Acylrest Ac entspricht der Teilformel R—CO—, worin R ein Wasserstoffatom oder ein Hydrocarbyl mit 1—23 Kohlenstoffatomen ist (der Acylrest R—CO—, worin R für Wasserstoff steht, ist Formyl).

Der Hydrocarbylrest R ist ein gesättigter oder ungesättigter acyclischer, carbocyclischer oder carbocyclisch-acyclischer Kohlenwasserstoffrest, der höchstens 23, vorzugsweise höchstens 17 Kohlenstoffatome hat. Er kann unsubstituiert oder substituiert sein und auch anstelle von einem, zwei oder mehreren C-Atomen Atome anderer Elemente (Heteroatome), wie insbesondere Sauerstoff, Schwefel und Stickstoff, aber auch z.B. phosphor und Silicium, tragen und somit, falls diese sich in einem cyclischen Rest befinden, einen heterocyclischen Rest (Heterocyclyl) oder einen heterocyclisch-acyclischen Rest bilden.

Als ungesättigte Reste sind solche bezeichnet, die eine oder mehrere Mehrfachbindungen, d.h. Doppel- und Dreifachbindungen, enthalten. Cyclische Reste, worin mindestens ein 6-gliedriger carbocyclischer oder 5- bis 8-gliedriger heterocyclischer Ring die maximale Anzahl nichtkumulierter

Doppelbindungen enthält, werden als aromatisch bezeichnet. Carbocyclische Reste, worin mindestes ein Ring als ein 6-gliedriger aromatischer Ring (d.h. Benzolring) vorliegt, werden als Arylreste bezeichnet.

Wenn nicht anders angegeben, enthalten in der vorliegenden Offenbarung mit "nieder", "mittellang" und "höher" bezeichnete acyclische Reste 1 bis 5, 6 bis 11 bzw. 12 bis 23 Kohlenstoffatome.

Ein acyclisches Hydrocarbyl ist beispielsweise ein gerader oder verzweigter aliphatischer Rest, der auch einfach oder mehrfach ungesättigt sein kann, z.B. Alkyl, Alkenyl, Alkadienyl, Alkatrienyl oder Alkynyl. Ein Alkyl ist beispielsweise ein Niederalkyl (wie Methyl, Ethyl, Propyl, Isopropyl, Butyl, Isobutyl, sec-Butyl, tert-Butyl, Pentyl oder Isopentyl), ein mittellanger Alkyl (wie Hexyl, Heptyl, Octyl, Nonyl, Decyl und Undecyl) oder ein höherer Alkyl (z.B. Tridecyl, Pentadecyl, Heptadecyl und Nonadecyl). Ein Alkenyl ist beispielsweise Vinyl, Allyl, Propenyl, Isopropenyl, Methallyl und 1-, 2- oder 3-Butenyl, auch 9-Decenyl sowie 8-Heptadecenyl; ein Alkadienyl ist beispielsweise 1,3-Butadienyl, 1,3- oder 2,4-Pentadienyl und 8,11-Heptadecadienyl; ein Alkatrienyl ist z.B. 8,11,14-Heptadecatrienyl, wobei in allen solchen Resten einzelne Doppelbindungen unabhängig voneinander in der cis-oder trans-Konfiguration stehen können. Ein Alkynyl ist z.B. Ethynyl, Propargyl und 1-, 2- oder 3-Butynyl.

Ein carbocyclischer Kohlenwasserstoffrest ist insbesondere ein mono-, bi- oder polycyclischer Cycloalkyl-, Cycloalkenyl- oder Cycloalkadienylrest, oder ein entsprechender aromatische Ringe enthaltender Arylrest. Bevorzugt sind Reste mit höchstens 12 Ringkohlenstoffatomen und 3- bis 8-, vorzugsweise 5- und/oder 6-gliedrigen Ringen, wobei sie auch einen oder mehrere acyclische Reste, z.B. die oben genannten, und insbesondere die Niederalkylreste, oder weitere carbocyclische Reste tragen können. Carbocyclisch-acyclische Reste sind solche, in welchen ein acyclischer Rest, insbesondere einer mit höchstens 7, vorzugsweise mit 1 bis 4 Kohlenstoffatomen, einen oder mehrere carbocyclische, gegebenenfalls aromatische Reste der obigen Definition trägt. Insbesondere sind Cycloalkyl-niederalkyl- und Arylniederalkylreste, sowie ihre im Ring und/oder Seitenkette ungesättigten Analogen, zu erwähnen.

Cycloalkyl ist z.B. Cyclopentyl und Cyclohexyl, sowie Bicyclo-[2,2,2]octyl, 2-Bicyclo[2,2,1]heptyl und Adamantyl, ferner auch 4,4-Dimethylcyclohexyl und 2,4,4,6-Tetramethylcyclohexyl; Cycloalkenyl ist z.B. einer der bereits genannten Cycloalkylreste, der eine Doppelbindung in 1-, 2- oder 3-Stellung trägt, wie 1-, 2- oder 3-Cyclopentenyl und 1-, 2- oder 3-Cyclohexenyl. Cycloalkyl-niederalkyl oder -niederalkenyl ist z.B. (Cyclopropyl-, Cyclopentyl- oder Cyclohexyl-) -methyl, -1- oder -2-ethyl bzw. -vinyl, -1-, -2- oder -3-propyl bzw. -allyl, ferner auch Dicyclohexylmethyl und Tricyclohexylmethyl; Cycloalkenyl-niederalkyl oder -niederalkenyl ist z.B. 1-, 2- oder 3-Cyclopentenyl- oder 1-, 2- oder 3-Cyclohexenyl-methyl, -1- oder -2-ethyl bzw. -vinyl, 1-, -2- oder -3-propyl bzw. -allyl.

Ein Arylrest ist in erster Linie ein Phenyl, ferner ein Naphthyl, wie 1- oder 2-Naphthyl, ein Biphenylyl, wie insbesondere 4-Biphenylyl, weiter auch ein Tolyl, wie o-, m- und p-Tolyl, und ein Xylyl.

Aryl-niederalkyl- und -niederalkenyl-Reste sind z.B. Phenyl-niederalkyl oder Phenyl-niederalkenyl, wie z.B. Benzyl, 1-oder 2-Phenylethyl, 1-, 2- oder 3-Phenylpropyl, Diphenylmethyl (d.h. Benzhydryl), Trityl und 1- oder 2-Napththylmethyl, bzw. Styryl oder Cinnamyl.

Heterocyclische Reste, einschliesslich der heterocyclisch-acyclischen Reste, sind insbesondere monocyclische, aber auch bi- oder polycyclische aza-, thia-, oxa-, thiaza-, thiadiaza-, oxaza-, diaza-, triaza- oder tetrazacyclische Reste aromatischen Charakters, ferner entsprechende partiell oder ganz gesättigte heterocyclische Reste dieser Art, wobei solche Reste gegebenenfalls, z.B. wie die obengenannten carbocyclischen oder Arylreste, weitere acyclische, carbocyclische oder heterocyclische Reste tragen können und mono-di- oder polysubstituiert sein können. Der acyclische Teil in heterocyclisch-acyclischen Resten hat z.B. die für die entsprechenden carbocyclisch-acyclischen Reste gegebene Bedeutung. In erster Linie sind es unsubstituierte oder substituierte monocyclische monoaza-, monothia- oder monooxacyclische Reste, wie Aziridinyl, Oxiranyl und Thiiranyl, und insbesondere heterocyclische Reste aromatischen Charakters, wie Pyrryl, z.B. 2-Pyrryl oder 3-Pyrryl, Pyridyl, z.B. 2-, 3- oder 4-Pyridyl, ferner Thienyl, z.B. 2- oder 3-Thienyl, oder Furyl, z.B. 2-Furyl; bicyclische monoaza- monooxa- oder monothiacyclische Reste, wie Indolyl, z.B. 2- oder 3-Indolyl, Chinolinyl, z.B. 2- oder 4-Chinolinyl, Isochinolinyl, z.B. 1-Isochinolinyl, Benzofuranyl, z.B. 2- oder 3-Benzofuranyl, oder Benzothienyl, z.B. 2- oder 3-Benzothienyl; monocyclische diaza-, triaza-, tetraza-, oxaza-, thiaza- oder thiadiazacyclische Reste, wie Imidazolyl, z.B. 2-Imidazolyl, Pyrimidinyl, z.B. 2- oder 4-Pyrimidinyl, Triazolyl, z.B. 1,2,4-Triazol-3-yl, Tetrazolyl, z.B. 1- oder 5-Tetrazolyl, Oxazolyl, z.B. 2-Oxazolyl, Isoxazolyl, z.B. 3- oder 4-Isoxazolyl, Thiazolyl, z.B. 2-Thiazolyl, Isothiazolyl, z.B. 3-oder 4-Isothiazolyl oder 1,2,4- oder 1,3,4-Thiadiazolyl, z.B. 1,2,4-Thiadiazol-3-yl oder 1,3,4-Thiadiazol-2-yl; oder bicyclische diaza-, oxaza-oder thiazacyclische Reste, wie Benzimidazolyl, z.B. 2-Benzimidazolyl, Benzoxazolyl, z.B. 2-Benzoxazolyl, oder Benzthiazolyl, z.B. 2-Benzthiazolyl. Entsprechende partiell oder ganz gesättigte Reste sind z.B. Tetrahydrothienyl, wie 2-Tetrahydrothienyl, Tetrahydrofuryl, wie 2-Tetrahydrofuryl, Pyrrolidinyl, wie 2-Pyrrolidinyl, Tetrahydropyridyl, wie 1,2,3,4- oder 1,2,3,6-Tetrahydropyrid-2-yl, oder Piperidyl, wie 2-, 3- oder 4-Piperidyl, ferner auch Morpholinyl, Thiomorpholinyl, Piperazinyl und 2-N,N'-Bis-niederalkylpiperazinyl, wie insbesondere 2-N,N'-Dimethylpiperazinyl. Diese Reste können auch einen oder mehrere acyclische, carbocyclische oder heterocyclische Reste, insbesondere die oben genannten, tragen. Hetercyclischacyclische Reste sind insbesondere von acyclischen Resten mit höchstens 7, vorzugsweise mit 1—4 Kohlenstoffatomen, z.B. von den oben genannten, abgeleitet und können einen, zwei oder mehrere heterocyclische Reste, z.B. die oben genannten, tragen.

Wie bereits erwähnt wurde, kann ein Hydrocarbyl (einschliesslich eines Heterocyclyls) durch einen,

zwei oder mehrere gleichartige oder verschiedenartige Substituenten, z.B. freie oder veretherte Hydroxylgruppen, Halogenatome (wie Chlor, Fluor oder Brom), Ketogruppen, sowie freie und funktionell abgewandelte Carboxylgruppen (z.B. Carbamoylgruppen und veresterte Carboxylgruppen) substituiert sein.

Eine im Hydrocarbyl als Substituent vorliegende veretherte Hydroxylgruppe ist z.B. eine gegebenenfalls durch Nitro, Niederalkoxy, Niederalkyl oder Halogene substituierte Phenoxy-, Benzyloxy- oder Phenethyloxygruppe und in erster Linie eine Niederalkoxygruppe, wie Methoxy-, Ethoxy-, Propoxy-, Isopropoxy-, Butoxy-, tert.-Butoxy-, Pentyloxy-, Hexyloxy- und Heptyloxy-Gruppe. Diese, insbesondere die linearen Niederalkoxygruppen können wiederum, insbesondere am endständigen C-Atom, durch eine Niederalkoxygruppe substituiert sein; solche Niederalkoxy-niederalkoxy-Gruppen sind z.B. Methoxy- oder Ethoxy-methoxy, 2-(Methoxy- oder Ethoxy)-ethoxy, 2-Butoxyethoxy 3-(Methoxy- oder Ethoxy)-propoxy und 4-(Methoxy- oder Ethoxy)-butoxy. Eine solche Veretherung kann sich mehrmals wiederholen, insbesondere in linearen Resten, wie im Spezialfall bei denjenigen, die von einfachen aliphatischen Glykolen, wie 1,4-Butandiol und vor allem Ethylenglykol, als wiederholenden Bausteinen abgeleitet sind und z.B. der Formel Alk-$(O—CH_2—CH_2—)_n$—O—, worin Alk ein Niederalkyl und n = 1 bis 4 ist entsprechen d.h. beispielsweise in der 2-[2-(Methoxy- oder Ethoxy)-ethoxy]-ethoxy- oder 2-{2-[2-(Methoxy- oder Ethoxy)-ethoxy]-ethoxy}-ethoxy-Gruppe. Derartige mehrfach veretherte Alkoxygruppen bezeichnet man einfach als lineare Mono-, Di-, Tri-oder Poly-oxaalkoxygruppen, d.h. solche Alkoxygruppen, in welchen eines bzw. 2, 3 oder mehrere nicht-benachbarte C-Atome durch O-Atome ersetzt sind, wie 2-Oxa-propoxy, 2- und 3-Oxa-butoxy, 3- und 4-Oxa-pentyloxy, 3-, 4- und 5-Oxa-hexyloxy, 3,6-Dioxa-heptyloxy, 3,6-Dioxaoctyloxy, 3,6,9-Trioxa-decyloxy und 3,6,9-Trioxa-undecyloxy.

Eine als Substituent im Hydrocarbyl vorliegende veresterte Carboxylgruppe ist insbesondere eine solche, in welcher das Wasserstoff durch einen der oben beschriebenen unsubstituierten Kohlenstoffreste, vor allem ein Niederalkyl oder Phenylniederalkyl ersetzt ist (z.B. Methoxycarbonyl, Ethoxycarbonyl und Benzyloxycarbonyl).

Bevorzugte Acylreste Ac leiten sich von unsubstituierten und substituierten Monocarbonsäuren und Dicarbonsäuren mit 1 bis 20 C-Atomen ab. Dazu gehören einerseits insbesondere Reste acyclischer Monocarbonsäuren, wie Alkanoylreste von Niederalkancarbonsäuren, (z.B. Formyl, Acetyl, Propionyl, Butyryl, Isobutyryl, Valeryl, Isovaleryl, Pivaloyl [2,2-Dimethylpropanoyl] und Hexanoyl [Caproyl]), von Alkan- und Alkensäuren mittlerer Länge (z.B. Heptanoyl, Octanoyl, Decanoyl, Undecanoyl, Lauroyl bzw. ein Alkenoyl, wie 10-Undecenoyl) und von höheren Alkancarbonsäuren (z.B. Myristoyl, Palmitoyl und Stearoyl) bzw. von ihren ungesättigten Analogen (Alkenoyl-, Alkadienoyl und Alkatrienoylreste, wie Oleoyl, Elaidoyl, Linoleoyl [9,12-Octadecadienoyl] bzw. Linolenoyl [9,12,15-Octadecatrienoyl]), wobei alle diese Reste auch substituierte Analoge mit den obengenannten Substituenten bilden können, beispielsweise halogenierte, wie chlorierte und bromierte Alkanoylreste (z.B. Chloracetyl, Bromacetyl und α-Bromisovaleryl), Hydroxyalkanoylreste und ihre veretherten Analoga (z.B. Lactoyl, Ricinoleoyl [d-12-Hydroxyoleoyl] bzw. 2-Methoxy- oder 2-Ethoxypropanoyl), Ketoalkanoylreste (z.B. Lävulinoyl), sowie gegebenenfalls veresterte ω-Carboxyalkanoylreste (insbesondere ω-Niederalkoxycarbonylalkanoylreste), d.h. monovalente Acylreste aliphatischer Dicarbonsäuren (z.B. der Bernstein-, Glutar-, Adipin-, Pimelin-, Suberin-, Azelain-, Sebacin- und Erucasäure), welche am endständigen Carboxyl gegebenenfalls durch Niederalkanole (wie Methanol, Ethanol oder tert-Butylalkohol) oder durch gegebenenfalls substituierte Benzylalkohole verestert sind. Zu bevorzugten Acylresten gehören auch Reste carbocyclischer und carbocyclisch-acyclischer Carbonsäuren, insbesondere monocyclische und bicyclische Arylcarbonyl- (Aroyl-)-Reste (z.B. Benzoyl, o-, m- und p-Toluoyl, und 1- oder 2-Naphthoyl) und araliphatische Reste, wie Aralkylcarbonyl ud Aralkenylcarbonyl (z.B. Phenylacetyl, 1- und 2-Phenylpropionyl, bzw. Cinnamoyl), welche alle im aromatischen Ring auch 1—3 gleiche oder verschiedene Substituenten tragen können, wie Nitro (z.B. 4-Nitrobenzoyl), Halogen, insbesondere Chlor oder Fluor (z.B. 4-Chlorbenzoyl und 4-Fluorbenzoyl), Hydroxyl (z.B. Salicyloyl oder 4-Hydroxybenzoyl), verethertes Hydroxyl, insbesondere Niederalkoxy, wie Methoxy (z.B. Anisoyl, Veratroyl und Piperonyloyl), und Carboxyl (z.B. Phthaloyl, Isophthaloyl und Terephthaloyl), insbesondere in veresterter Form, z.B. als Niederalkoxycarbonyl, wie Methoxycarbonyl oder Ethoxycarbonyl. Zu bevorzugten Acylresten heterocyclischer Carbonsäuren gehören beispielsweise 2-Furoyl, 2-Thenoyl, Pyrrol-2-carbonyl, Nicotinoyl und Isonicotinoyl.

In einem von einem Kohlensäuremonoester abgeleiteten Oxycarbonylrest Cb ist eine der beiden Hydroxylgruppen der Kohlensäure durch ein 1—24 C-Atome aufweisendes Hydrocarbyl, dessen freie Valenz von einem gesättigten Kohlenstoffatom ausgeht, verestert. Eine derartige veresterte Oxycarbonylgruppe ist insbesondere eine der Teilformel R—$CH_2$—O—CO—, worin R Wasserstoff ist oder eine beliebige der oben für Hydrocarbyl angeführten Bedeutungen haben kann.

Bevorzugte Bedeutungen der O-acylierenden Oxycarbonylreste Cb sind z.B. diejenigen der Teilformel R—$CH_2$—O—CO—, worin R ein Cycloalkyl mit 5—7 Ringgliedern, ein gegebenenfalls durch Halogen (insbesondere Chlor), $C_{1-4}$-Alkyl (insbesondere Methyl), $Cl_{1-4}$-Alkoxyl (insbesondere Methoxy), Methylendioxy und/oder Nitro einfach oder mehrfach im Ring substituiertes Phenyl oder Phenylmethyl, oder insbesondere ein linearer aliphatischer Rest ist, vorzugsweise einer mit ungerader Anzahl der C-Atome. Besonders bevorzugt als R sind derartige Alkenyl- und speziell Alkylreste mittlerer Länge.

Besonders bevorzugt sind auch analoge Reste, die am endständigen C-Atom durch veretherte Hydroxylgruppen, vor allem die oben charakterisierten und hervorgehobenen, substituiert sind,

insbesondere solche worin in der Kohlenstoffkette eines linearen Alkyls eines oder mehrere C-Atome durch O-Atome ersetzt sind. (Wenn mehrere O-Atome vorhanden sind, so sind sie jeweils durch mindestens ein C-Atom, vorzugsweise aber durch mehrere, vor allem 2, C-Atome voneinander getrennt.) Als Beispiel derartiger Alkoxycarbonylreste in der Bedeutung von Cb sind zu nennen: 2-Methoxy-ethoxycarbonyl (3-Oxabutoxycarbonyl), 2-Ethoxy-ethoxycarbonyl (3-Oxapentyloxycarbonyl), 2-(2-Methoxy-ethoxy)-ethoxycarbonyl (3,6-Dioxaheptyloxycarbonyl), 2-(2-Ethoxy-ethoxy)-ethoxycarbonyl (3,6-Dioxaoctyloxycarbonyl), 2-[2-(2-Methoxyethoxy)-ethoxy]-ethoxycarbonyl (3,6,9-Trioxadecyloxycarbonyl), 4-Methoxy-butoxycarbonyl (5-Oxahexyloxycarbonyl), 4-Ethoxy-butoxycarbonyl (5-Oxaheptyloxycarbonyl) und 4-(2-Methoxy-ethoxy)-butoxycarbonyl (5,8-Dioxanonyloxycarbonyl).

Auch für den Rest Cb als Substituent der endständigen Aminogruppe kommen alle diese bevorzugten Bedeutungen besonders in Betracht, zusätzlich jedoch sind auch solche hervorzuheben, die durch das Symbol $Cb_o$ sowie die Teilformel $R_o$—O—CO— gekennzeichnet sind, worin $R_o$ ein neutral und/oder acidolytisch abspaltbares Hydrocarbyl ist; diese Reste können zugleich als Aminoschutzgruppen klassifiziert werden.

Ein solches Hydrocarbyl $R_o$ ist z.B. ein mono- oder bicyclisches α-Arylniederalkyl $R_a$ (d.h. ein solches, worin das Aryl und das Sauerstoffatom der Oxycarbonylgruppe an dasselbe Kohlenstoffatom des Niederalkyls gebunden sind, insbesondere ein α-Phenylniederalkyl, in erster Linie Benzyl. Entsprechende Acylreste $R_a$—O—CO— sind beispielsweise gegebenenfalls im aromatischen Ring durch Halogenatome, Nitrogruppen, Niederalkyl- oder Niederalkoxygruppen substituierte Benzyloxycarbonylgruppen, wie unsubstituiertes Benzyloxycarbonyl (d.h. Carbobenzoxy), p-Brom- oder p-Chlorbenzyloxycarbonyl, p-Nitrobenzyloxycarbonyl, p-Methoxybenzyloxycarbonyl und p-Tolyloxycarbonyl, sowie auch 2-(4-Biphenylyl)-2-propyloxycarbonyl und ähnliche im Schweizer Patent 509 266 beschriebene Aralkoxycarbonylreste. Diese Reste lassen sich, wie noch weiter unten näher beschrieben wird, unter neutralen Bedingungen hydrogenolytisch, oder aber vorzugsweise acidolytisc abspalten.

Ein weiteres solches Hydrocarbyl $R_o$ ist ein sekundäres oder, vorzugsweise, tertiäres Alkyl oder Cycloalkyl $R_b$, insbesondere ein solches mit höchstens 12 C-Atomen, wie sie auch oben genannt wurden. Ein entsprechender Acylrest $R_b$—O—CO— ist beispielsweise vor allem das tert-Butoxycarbonyl, oder auch ein analoger Rest, wie Isopropyloxycarbonyl, tert-Amyloxycarbonyl (d.h. 1,1-Dimethylpropyloxycarbonyl), Diisopropylmethoxycarbonyl, Cyclopentyloxycarbonyl, Cyclohexyloxycarbonyl, d-Isobornyloxycarbonyl und Adamamtyloxycarbonyl. Diese Reste lassen sich, wie noch weiter unten näher beschrieben wird, vornehmlich unter sauren Bedingungen (acidolytisch) abspalten.

Ein noch weiteres Hydrocarbyl $R_o$ ist ein β-(Trihydrocarbylsilyl)-ethoxycarbonylrest $R_c$, der in β-Stellung eine mit drei unsubstituierten $C_{1-6}$-Kohlenwasserstoffresten substituierte Silylgruppe, wie Triphenylsilyl-, Dimethyl-butyl-silyl- oder vor allem Trimethylsilylgruppe, trägt. Entsprechende Acylreste $R_c$—O—CO—, d.h. β-(Trihydrocarbylsilyl)-ethoxycarbonylreste, wie ein β-(Triniederalkylsilyl)-ethoxycarbonyl, z.B. insbesondere β-(Trimethylsilyl)-ethoxycarbonyl, bilden mit der zu schützenden Aminogruppe entsprechende β-Trihydrocarbylsilyl-ethoxycarbonylaminogruppen (z.B. die β-Trimethylsilylethoxycarbonylaminogruppe). Sie sind unter den Bedingungen der sauren Hydrolyse und der Hydrogenolyse zwar beständig, aber lassen sich unter ganz spezifischen, sehr milden Bedingungen durch Einwirkung von Fluoridionen abspalten, wie noch weiter unten näher beschrieben wird.

Als Hydrocarbyl $R_o$ ist auch Allyl hervorzuheben. Das entsprechende Allyloxycarbonyl lässt sich nicht nur acidolytisch, sondern besonders auch unter sehr milden neutralen Bedingungen mit Dimedon oder durch die spezifische reduktive Einwirkung von Tributylzinnhydrid unter Katalyse mit Palladium-(O)-tetrakis-(triphenylphosphin)-Komplex abspalten.

Aminoschutzgruppen, welche bei der Synthese der Peptidkette verwendet werden, sind einschliesslich entsprechender Abspaltungsmethoden in Uebersichtsreferaten und Nachschlagewerken, wie Houben-Weyl: Methoden der organischen Chemie; 4. Auflage, Band 15/I und II, E. Wünsch (Herausgeber): Synthese von Peptiden (Georg-Thieme Verlag, Stuttgart; 1974), ausführlich beschrieben. Vorzugsweise werden acidolytisch und/oder neutral abspaltbare Aminoschutzgruppen verwendet.

Die engere Auswahl der Schutzgruppe X richtet sich nach den jeweiligen Eigenschaften der Acylgruppen Ac bzw. Oxycarbonylgruppen Cb, insbesondere nach ihrer Stabilität unter den Reaktionsbedingungen für die Abspaltung der Schutzgruppe.

Salze von Verbindungen der obigen Formel I mit salzbildenden Eigenschaften leiten sich in erster Linie von denjenigen ab, worin B Wasserstoff bedeutet, und sind Säureadditionssalze, insbesondere pharmazeutisch verwendbare, nicht-toxische Säureadditionssalze mit anorganischen Säuren, z.B. Chlorwasserstoffsäure, Bromwasserstoffsäure, Schwefelsäure oder Phosphorsäuren, oder mit organischen Säuren, wie Sulfonsäuren, wie aromatischen Sulfonsäuren, z.B, Benzolsulfonsäure, p-Toluolsulfonsäure oder Naphthalin-2-sulfonsäure, oder insbesondere aliphatischen Sulfonsäuren, z.B, Methansulfonsäure, Ethansulfonsäure, 2-Hydroxyethansulfonsäure und Ethan-1,2-disulfonsäure, sowie auch Carbonsäuren, z.B. Essigsäure, Propionsäure, Glykolsäure, Bernsteinsäure, Maleinsäure, Hydroxymaleinsäure, Benzoesäure, Zimtsäure, Mandelsäure, Salicylsäure, 4-Aminosalicylsäure, 2-Phenoxybenzoesäure, 2-Acetoxy-benzoesäure, Embonsäure, Nicotinsäure oder Isonicotinsäure.

Infolge der engen Beziehung zwischen den neuen Verbindungen in freier Form und in Form ihrer Salze, inkl. auch solcher Säureadditionssalze, die als Zwischenprodukte, z.B. bei der Reinigung der neuen Verbindungen oder zu ihrer Identifikation verwendet werden können, sind vorausgehend und nachfolgend

unter den freien Verbindungen sinn- und zweckgemäss gegebenenfalls auch die entsprechenden Salze zu verstehen.

Die Verbindungen der vorliegenden Erfindung weisen wertvolle Eigenschaften, in erster Linie pharmakologische Wirkungen auf, indem sie eine physiologische Wirkung zeigen, die im Grundcharakter der Wirkung des Desferrioxamins B ähnlich ist. Sie können deshalb in ähnlichen therapeutischen Indikationen wie dieses, jedoch mit dem wesentlichen Vorteil der oralen Verabreichung, angewendet werden, z.B. insbesondere zur Behandlung von Funktionsstörungen, in denen die Konzentration des dreiwertigen Eisens ($Fe^{3+}$-Ions) in Körperzellen übernormal hoch ist, wie bei Hämochromatose und Hämosiderose. Indem sie überdies noch in ähnlicher Weise auch Aluminium-Ionen, wie. z.B. bei Dialyse-Encephalopathie, Osteomalacia und der Alzheimer-Krankheit, binden, können sie auch in diesen Indikationsbereichen mit Erfolg eingesetzt werden.

Besonders bevorzugte Verbindungen der Formel I für diese Anwendung sind insbesondere diejenigen, worin $AA^1$, $AA^2$ und $AA^3$ alle dieselbe Bedeutung von Cb, insbesondere eine der oben hervorgehobenen, haben, wobei B auch eine dieser Bedeutungen von Cb, vorzugsweise eine identische aufweisen kann, aber vor allem für Wasserstoff steht. Als solche besonders bevorzugte Oxycarbonylreste Cb sind beispielsweise Alkoxycarbonylreste, deren Kohlenstoffkette durch 1,2 oder mehrere O-Atome unterbrochen sein kann, zu nennen, d.h. Niederalkoxycarbonyl-Reste (wie Methoxy-, Ethoxy-, Propoxy-, Isopropoxy-, Butoxy-, i-Butoxy-, sec-Butoxy-, tert-Butoxy-, Pentyloxy-, Isopentyloxy- und Hexyloxy-carbonyl), mittellange Alkoxycarbonyl- und Alkylenoxycarbonyl-Reste (wie Heptyloxy-, Octyloxy-, Nonyloxy-, Decyloxy- und Undecyloxy-, bzw. 10-Undecenyloxy-carbonyl), sowie höhere Alkoxycarbonyl-Reste und analoge ein-, zwei- oder mehrfach ungesättigte Reste (wie insbesondere Dodecyloxy-, Tetradecyloxy-, Hexadecyloxy- und Octadecyloxy-, bzw. cis- oder trans-9-Octadecenyloxy-, -9,12-Octadecadienyloxy-und -9,12,15-Octadecatrienyloxycarbonyl), ferner auch Benzyloxy-, 2-Phenylethoxy- und Cinnamyloxy-carbonyl, welche alle im Ring in der oben hervorgehobenen Weise substituiert sein können. Als Beispiel solcher Verbindungen sind zu erwähnen: N,O,O',O''-Tetra-(ethoxycarbonyl)-desferrioxamin B und O,O',O''-Tri-(ethoxycarbonyl)-desferrioxamin B, N,O,O',O''-Tetra(propoxycarbonyl)-desferrioxamin B und O,O',O''-Tri-(propoxycarbonl)-desferrioxamin B, N,O,O',O''-Tetra-(butoxycarbonyl)-desferrioxamin B und O,O',O''-Tri-(butoxycarbonyl)-desferrioxamin B, N,O,O',O''-Tetra-(3-oxabutoxycarbonyl)-desferrioxamin B und O,O',O''-Tri-(3-oxa-butoxycarbonyl)-desferrioxamin B, N,O,O',O''-Tetra-(hexyloxycarbonyl)-desferrioxamin B und O,O',O''-Tri-(hexyloxycarbonyl)-desferrioxamin B, N,O,O',O''-Tetra-(3,6-dioxaheptyloxycarbonyl)-desferrioxamin B und O,O',O''-Tri-(3,6-dioxaheptyloxycarbonyl)-dessferrioxamin B, N,O,O',O''-Tetra-(octyloxycarbonyl)-desfereioxamin B und O,O',O''-Tri-(octyloxycarbonyl)-desferrioxamin B,N,O,O',O''-Tetra-(3,6,9-trioxadecyloxycarbonyl)-desferrioxamin B, und O,O',O''-Tri-(3,6,9-trioxadecyloxycarbonyl)-desferrioxamin B, N,O,O',O''-Tetra-(10-undecenyloxycarbonyl)-desferrioxamin B, und O,O',O''-Tri-(10-undecenyloxycarbonyl)-desferrioxamin B, N,O,O',O''-Tetra-(dodecyloxycarbonyl)-desferrioxamin B und O,O',O''-Tri-(dodecyloxyarbonyl)-desferrioxamin B, sowie N,O,O',O''-Tetra(octadecyloxycarbonyl)-desferrioxamin B und O,O',O''-Tri-(octadecyloxycarbonyl)-desferrioxamin B, ferner auch N-tert-Butoxycarbonyl-O,O',O''-tri-(butoxycarbonyl)-desferrioxamin B, N-tert-Butoxycarbonyl-O,O',O''-tri-(3-oxabutoxycarbonyl)-desferrioxamin B, N-tert-Butoxycarbonyl-O,O',O''-tri-(hexyloxycarbonyl)-desferrioxamin B, N tert-Butoxycarbonyl-O,O',O''-tri-(3,5-dioxaheptyloxycarbonyl)-desferrioxamin B und N-tert-Butoxycarbonyl-O,O',O''-tri-(octyloxycarbonyl)-desferrioxamin B.

Für die therapeutische Anwendung kommen auch gemischte Acylate der Formel I in Betracht, insbesondere solche, worin B für Wasserstoff oder Cb steht und $AA^1$, $AA^2$ und $AA^3$ die Bedeutungen von Cb haben, die aber nicht für alle Symbole gleich ist. Unter diesen gemischten O,O',O''-Triacylaten (und auch N,O,O',O''-Tetraacylaten) sind insbesondere solche bevorzugt, in welchen den Oxycarbonylresten Cb einer der oben besonders hervorgehobenen unsubstituierten linearen aliphatischen Hydrocarbylreste zugrunde liegt, wobei mindestens einer davon ein mittellanger oder höherer aliphatischer Hydrocarbylrest ist. (Diese besonders bevorzugten Hydrocarbylreste sind analog den Acylresten der in der Natur vorkommenden Fettsäuren, anstelle des Carbonyls weisen sie aber Methylen auf.) Ueblicherweise kommen jedoch solche gemischten Acylate als Bestandteil praktisch untrennbarer Gemische mit anderen Isomeren vor, in welchen jeder der 3 Oxycarbonylreste Cb jede beliebige der 3 Hydroxylgruppen verestern kann. Dementsprechend ist die Bezeichnung ein "O,O',O''-($Cb^1$, $Cb^2$, $Cb^3$)-Desferrioxamin B", wie beispielsweise O,O',O''-(Tetradecyloxycarbonyl, Hexadecyloxycarbonyl, Octadecyloxycarbonyl)-, O,O',O''-(Dibutoxycarbonyl, Octyloxycarbonyl)-, O,O',O''-(Diethoxycarbonyl, cis-9-Octadecenyloxycarbonyl)- und O,O',O''-(Pentyloxycarbonyl, Di-10-undecenyloxycarbonyl)-desferrioxamin B, nicht nur als Bezeichnung für eine individuelle Verbindung mit unbestimmter Zuordnung der Acylgruppen zu einzelnen O-Atomen zu verstehen, sondern als Sammelbegriff für das ganze Isomerengemisch enthaltend die Gesamtheit isomerer Verbindungen mit beliebiger Kombination von O, O', und O'' mit $Cb^1$, $Cb^2$ und $Cb^3$ auszulegen. Analog kombinierte Polyacylate und ihre Isomerengemische kommen sehr häufig in der Natur, z.B. als Wachse und Fette, speziell Triglyzeride, vor, und es ist bekannt, dass es infolge der prinzipiellen Aehnlichkeit der Fettsäuren untereinander für die gesamten Eigenschaften eines Acylats praktisch belanglos ist, ob ein spezifisches Hydroxyl mit diesem oder jenem Acylrest der gegebenen Auswahl verestert ist. Einzelne Isomere sind in ihrem physikalischen, chemischen und biologischen Verhalten so ähnlich, dass die Eigenschaften eines solchen Isomerengemisches in der Regel dem Durchschnittswert von Einzelbeiträgen individueller Komponenten (Acylreste) entsprechen. Die Einzelkomponenten eines solchen

Gemisches, sowie, nach Hydrolyse, die einzelnen Acylreste kann man durch moderne analytische Trennmethoden, wie Gaschromatographie (GLC) oder Hochdruckflüssigkeitschromatographie (HPLC) qualitativ und quantitativ analytisch erfassen. Zur allgemeinen Charakterisierung genügen jedoch oft klassische analytische Methoden, wie sie z.B. in der Technologie der Fettverarbeitung geläufig sind, wie die Bestimmung der Säurezahl, Verseifungszahl, Hydrierzahl oder Jodzahl usw.

Die Verbindungen der vorliegenden Erfindung können auch als wertvolle Zwischenprodukte zur Herstellung anderer therapeutisch wertvoller Verbindungen dienen.

Besonders bevorzugt für diese Anwendung sind z.B. diejenigen der Verbindungen der Formel I, worin B für einen als Aminoschutzgruppen anwendbaren, leicht abspaltbaren Oxycarbonylrest $Cb_o$ steht und $AA^1$, $AA^2$ und $AA^3$, die voneinander verschieden oder vorzugsweise alle gleich sein können, Acylreste Ac sowie auch veresterte Oxycarbonylreste Cb der obengenannten allgemeinen und besonders hervorgehobenen Bedeutungen darstellen. Vor allem sind solche Verbindungen bevorzugt, in welchen $Cb_o$ eine der oben besonders hervorgehobenen Bedeutungen hat und in erster Linie das tert-Butoxycarbonyl (BOC), das 2-(Trimethylsilyl)-ethoxycarbonyl oder das Allyloxycarbonyl ist. Sofern sie Acylreste Ac tragen, sind diese Verbindungen wichtige Ausgangsstoffe für O,O',O''-Triacylate Desferrioxamins B mit freier endständiger Aminogruppe, in welche sie durch konventionelles Abspalten der Aminoschutzgruppe $Cb_o$ umgewandelt werden können. Für diesen Zweck kommen vor allem diejenigen in Betracht, worin $AA^1$, $AA^2$ und $AA^3$ je für denselben Acylrest, insbesondere einen der obengenannten, der sich von einer unsubstituierten, vorzugsweise gesättigten, aliphatischen Monocarbonsäure mit höchstens 18 C-Atomen ableitet, stehen, in erster Linie diejenigen, worin $AA^1$, $AA^2$ und $AA^3$ je für Acetyl, Butyryl, Pivaloyl, Hexanoyl, Octanoyl, Palmitoyl oder Stearoyl stehen, sowie auch diejenigen worin $AA^1$, $AA^2$ und $AA^3$ je für ein monocyclisches Aroyl oder Arylniederalkanoyl, vor allem Benzoyl und Phenylacetyl, steht, wobei diese im Ring durch Methyl, Methoxy, Chlor und/oder Nitro substituiert sein können, aber vorzugsweise unsubstituiert sind.

Besonders bevorzugt sind die in den Beispielen genannten Verbindungen.

Erfindungsgemäss werden Verbindungen der Formel I und Salze solcher Verbindungen mit salzbildenden Eigenschaften unter Anwendung konventioneller, z.B. aus der Peptidchemie allgemein bekannter Analogieverfahren hergestellt, indem man

a) eine Verbindung der Formel

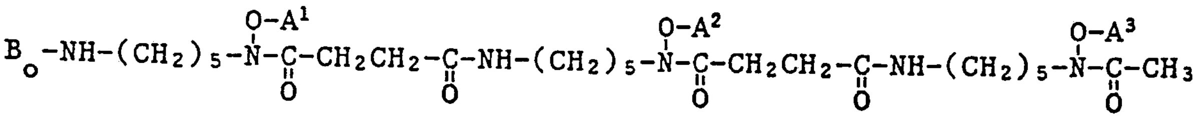

(II)

worin $A^1$, $A^2$ und $A^3$, jedes unabhängig, Wasserstoff ist oder eine der oben definierten Bedeutungen von Ac hat und $B_o$ Wasserstoff ist oder, falls mindestens eines der Symbole $A^1$, $A^2$ und $A^3$ Wasserstoff ist, auch eine von einer Oxycarbonylgruppe unterschiedliche Aminoschutzgruppe $X_o$ sein kann, mit einem vom oben definierten Rest Cb abgeleiteten reaktiven Derivat eines Kohlensäuremonoesters acyliert, oder

b) eine Verbindung der Formel I, worin B für einen von Ethoxycarbonyl oder Benzyloxycarbonyl unterschiedlichen Rest Cb und $AA^1$, $AA^2$ und $AA^3$ für Wasserstoff stehen, zu einer Verbindung der Formel I, worin B für einen von Ethoxycarbonyl oder Benzyloxycarbonyl unterschiedlichen Rest Cb steht und $AA^1$, $AA^2$ und $AA^3$ unabhängig voneinander die Bedeutung von Ac und/oder Cb haben, acyliert, oder

c) eine Verbindung der Formel I, worin mindestens eines der Symbole $AA^1$, $AA^2$ und $AA^3$ eine der Bedeutungen von Cb hat und B für eine Aminoschutzgruppe X steht, durch Abspalten dieser Aminoschutzgruppe in eine entsprechende Verbindung der Formel I, worin B Wasserstoff ist, oder in ein Salz davon umwandelt, und/oder, gewünschtenfalls, eine freie Verbindung der Formel I mit salzbildenden Eigenschaften in ein Salz umwandelt und/oder eine Verbindung der Formel I aus einem solchen Salz freisetzt.

Die erfindungsgemässe Acylierung der oben definierten Ausgangsstoffe der Formel II, insbesondere des Desferrioxamins B oder eines an der Aminogruppe geschützten Derivats davon, wird in an sich bekannter Weise unter Anwendung konventioneller allgemeiner Methoden durchgeführt, wobei man durch eine geeignete Wahl der Acylierungsmittel und Reaktionsbedingungen das Ausmass der Substitution im Bezug auf Anzahl und Art der eingeführten Acylreste weitgehend steuern kann.

Wenn man beispielsweise zur erfindungsgemässen Acylierung als das reaktive Kohlensäurederivat ein symmetrisches Anhydrid (d.h. ein Dicarbonat) der Formel Cb—O—Cb, oder vorzugsweise ein unsymmetrisches Anhydrid mit einer anorganischen Säure, in erster Linie ein Kohlensäuremonohalogenid, wie vor allem einen Chlorameisensäureester der Formel CbC1 verwendet und in Gegenwart eines stark basischen säurebindenden Mittels arbeitet, erhält man üblicherweise Produkte der Formel I, welche die Oxycarbonylgruppen Cb sowohl am N- wie auch an den O-Atomen tragen. Die genannten Acylierungsmittel werden dabei üblicherweise unverdünnt, oder aber gelöst in einer minimalen Menge eines inerten Lösungsmittels, wie eines chlorierten Kohlenwasserstoffs (z.B. Chloroform oder Dichlormethan) eingesetzt, die Reaktionstemperatur liegt üblicherweise zwischen etwa 0 bis etwa 50°C vornehmlich in der Umgebung der Raumtemperatur oder, leicht oberhalb davon. Die Umsetzung erfolgt unter basischen Bedingungen im pH-Bereich von etwa 8 bis etwa 12, vornehmlich zwischen etwa 8,5 bis

etwa 9,5 unter Anwendung von starken Basen; als solche kann man anorganische Basen, wie Alkalimetallcarbonate und insbesondere Alkalimetallhydroxide (z.B. Soda und Pottasche bzw. Natrium- oder Kalium-hydroxid), vornehmlich in einer wässrigen Lösung, wie einer etwa 2N- bis etwa 8N-, vorzugsweise 4N- bis 6N-Lösung, einsetzen, die man dem Reaktionsgemisch im gleichen Schritt mit dem Acylierungsmittel unter Einhalten des oben angegebenen erforderlichen pH-Bereichs portionenweise zufügt. Ein grösserer Ueberschuss am alkalischen Mittel, sowie Ueberschreiten des pH über etwa 10, ist insbesondere während längerer Zeitabschnitte zu vermeiden. Zum Einhalten der erforderlichen Basizität können auch starke organische Basen, wie insbesondere das 1,8-Diazabicyclo[5.4.0]undec-7-en und ähnliche cyclische Basen, angewendet werden, die den Vorteil vorweisen, dass einerseits die Reaktion meistenfalls in einem homogenen Reaktionsmedium verlaufen kann, andererseits ein vorübergehender Ueberschuss an Base nicht nachteilig ist, da derartige Basen weder das Acylierungsmittel noch die Reaktionsprodukte beeinträchtigen. Zur Acylierung mit überschüssigen Chlorameisensäureestern sind als die organische Base auch tertiäre Amine, z.B. Triethylamin, Ethyldiisopropylamin, Tributylamin, N,N-Dimethyl- und N,N-Diethylanilin, N-Methyl- und N-Ethyl-piperidin oder -morpholin und N,N'-Dimethylpiperazin, gut geeignet. — Unter Einhaltung obiger Bedingungen werden nebst der endständigen Aminogruppe auch sämtliche freie Hydroxylgruppen des Ausgangsstoffes acyliert. Vorzugsweise werden einheitliche N,O,O',O''-Tetraacylate bzw. O,O',O''-Triacylate hergestellt, d.h. Verbindungen der Formel I, worin $AA^1$, $AA^2$ und $AA^3$, und gegebenenfalls auch B, alle denselben Oxycarbonylrest Cb darstellen. In diesem Fall wird die Acylierung unter den obigen allgemeinen Bedingungen mit der äquimolaren Menge von 4 bzw. 3 Aequivalenten (oder einem kleineren Ueberschuss) eines einheitlichen Acylierungsmittels vorgenommen.

Wird jedoch ein gemischtes Tetra- oder Triacylat erwünscht, in welchem insbesondere $AA^1$, $AA^2$ und $AA^3$ nicht alle denselben Acylrest bedeuten, sondern verschiedene Bedeutungen von Cb haben, geht man beispielsweise so vor, dass man mit einem Gemisch von mehreren gleichartigen Acylierungsmitteln (z.B. Chloriden), in welchen 2 oder 3 verschiedene Acylreste in äquimolarem Verhältnis vertreten sind, umsetzt, z.B. mit einem Gemisch von Chlorameisensäureestern mit linearen aliphatischen Resten verschiedener Länge, wie den oben hervorgehobenen. Alternativ kann man auch mit äquivalenten Mengen individueller Acylierungsmittel (z.B. der oben genannten) separat nacheinander umsetzen, wobei man die Reaktion üblicherweise ohne Isolierung von teilweise acylierten Zwischenprodukten fortsetzt. Bei beiden Verfahrensvarianten resultiert als Produkt ein Gemisch isomerer Triacylate, in welchen man die einzelnen Oxycarbonylreste keinem spezifischen der 3 O-Atome zuordnen kann; in der Regel wird ein derartiges Gemisch als solches ohne Trennung individueller Komponenten angewendet. Wie bereits oben diskutiert, leisten einzelne Oxycarbonylreste ihren individuellen strukturellen Beitrag zu den allgemeinen chemischen und biologischen Eigenschaften eines Acylats praktisch unabhängig davon, an welchem spezifischen O-Atom sie lokalisiert sind, dementsprechend werden die Eigenschaften eines gemischten Triacylats, sowie auch eines Isomerengemisches solcher gemischten Triacylate, weitgehend dem statistischen Durchschnitt der Einzelbeiträge der beteiligten Acylreste entsprechen. Gemischte N,O,O',O''-Tetraacylate und insbesondere O,O',O''-Triacylate der Formel I, sowie deren Isomerengemische, haben demzufolge dieselben Anwendungsmöglichkeiten, sei es als Zwischenprodukte oder als therapeutische Wirkstoffe, wie die analogen Triacylate mit einheitlichen Oxycarbonylresten. Sinngemäss können diese gemischten Tetra- und Triacylate, sofern sie mindestens einen Oxycarbonylrest enthalten, anstelle der Reste Cb auch Carbonsäure-Acylreste Ac der oben angegebenen Bedeutung enthalten.

Durch geeignete Wahl der Reaktionsbedingungen kann man die erfindungsgemässe Acylierung auch so lenken, dass man vorwiegend, oder sogar praktisch ausschliesslich, eine selektive Acylierung der endständigen Aminogruppe erreicht. Vornehmlich geht man bei dieser Variante von Desferrioxamin B oder einem seiner Säureadditionssalzen aus und erhält man ein Zwischenprodukt der Formel I, worin B für Cb und $AA^1$, $AA^2$ und $AA^3$ jedes für Wasserstoff steht. (Derartige Verbindungen, insbesondere solche, worin B ein als Aminoschutzgruppe anwendbares Oxycarbonyl $Cb_o$ darstellt, sind vorteilhafte Zwischenprodukte für weitere Umwandlung, insbesondere Acylierung, der freien Hydroxylgruppen, gegebenenfalls mit anschliessender Abspaltung der Schutzgruppe $Cb_o$). Bei vorsichtiger Arbeitsweise, insbesondere beim Einhalten von niedrigeren Temperaturen (vorzugsweise im Bereich von 0°C bis Raumtemperatur) und kurzer Reaktionszeit, erreicht man eine zufriedenstellende selektive N-Acylierung auch mit den oben erwähnten energischen reaktiven Kohlensäurederivaten, wie Halogeniden (insbesondere Chlorameisensäureestern) oder symmetrischen Anhydriden (Dicarbonaten, z.B. dem Di-tert-butyl-dicarbonat der Formel $t—C_4H_9—O—CO—O—CO—O—t—C_4H_9$), insbesondere unter Anwendung von einer knappen äquivalenten Menge des Acylierungsmittels oder einem geringen Ueberschuss davon. Gewünschtenfalls kann man eine noch höhere Selektivität erreichen, wenn man Aktivester der Formel $R_o—O—CO—Z$, worin $R_o$ die obengenannte Bedeutung hat und Z eine Aktivierungsgruppe, wie ein gegebenenfalls substituierter (z.B. durch Methyl, Methoxy, Halogene und insbesondere Nitro, wie insbesondere 4-Nitro) Phenoxyrest als das reaktive Kohlensäurederivat einsetzt. Ungeachtet des angewendeten Acylierungsmittels wird die Acylierung bei einer schwachen Basizität durchgeführt, d.h. in Gegenwart eines säurebindenden Mittels, dessen Basizität ausreicht, die zu acylierende endständige Aminogruppe in nicht-protonisiertem Zustand zu halten. Geeignete Mittel dieser Art sind nicht-acylierbare organische Basen, wie tertiäre Amine (z.B. die oben genannten), sowie insbesondere stickstoffhaltige heteroaromatische Basen (z.B. Chinolin, Collidin und vor allem Pyridin).

Sämtliche oben diskutierte Varianten des erfindungsgemässen Acylierungsverfahrens werden in inerten organischen Lösungsmitteln oder aber in wässrigem Milieu durchgeführt; zum besseren Vermischen der verschiedenen, oft spärlich löslichen oder miteinander mischbaren Komponenten arbeitet man vorzugsweise unter intensivem Rühren und mit Zusatz von inerten, mit Wasser mischbaren organischen Lösungsmitteln oder ihren zweckmässigen Gemischen, wie cyclischen Ethern (z.B. Dioxan oder Tetrahydrofuran), tertiären Amiden (z.B. N,N-Dimethylformamid, N,N-Dimethylacetamid, N-Methylpyrrolidon und Hexamethylphosphortriamid), Dimethylsulfoxid, Acetonitril, sowie auch tertiären Aminen (z.B. den obengenannten) oder Acetonitril oder ähnlichen Niederalkylcyaniden.

Die erfindungsgemässe nachträgliche O-Acylierung, d.h. die Umwandlung der Zwischenprodukte der Formel I, worin mindestens eines der Symbole Wasserstoff darstellt, in solche, worin diese Symbole für Ac oder Cb stehen, wird bei analoger Arbeitsweise und unter denselben Reaktionsbedingungen wie die oben geschilderte energische Acylierungsvariante (einschliesslich der gemischten Acylierungen) durchgeführt; sinngemäss verwendet man jedoch als Acylierungsmittel nicht nur die vom Rest Cb abgeleiteten, oben charakterisierten unsymmetrischen und symmetrischen Anhydride von Kohlensäuremonoestern, sondern auch entsprechende, vom Rest Ac abgeleitete reaktive Derivate von Carbonsäuren, wie insbesondere symmetrische Anhydride der Formel Ac—O—Ac, oder vorzugsweise unsymmetrische Anhydride mit anorganischen Säuren, in erster Linie Acylhalogenide, insbesondere Acylchloride, der Formel AcY, worin Ac die eingangs angegebenen Bedeutungen hat und Y Brom oder vorzugsweise Chlor ist. Im Spezialfall der Ameisensäure wird ihr gemischtes Anhydrid mit Essigsäure oder insbesondere mit Trifluoressigsäure bevorzugt. Vornehmlich werden dieser Umwandlung Produkte der selektiven N-Acylierung, vor allem Zwischenprodukte der Formel I, worin $AA^1$, $AA^2$ und $AA^3$ alle Wasserstoff bedeuten und B für Cb, insbesondere $Cb_0$ steht, unterworfen, um die oben erwähnten wertvollen O,O′,O″ -Triacylate herzustellen.

Zwischenprodukte der Formel I, worin $AA^1$, $AA^2$ und $AA^3$ alle Wasserstoff bedeuten, können auch durch nachträgliche Abspaltung der O-Acylreste durch Ammonolyse erhalten werden, durch welche man ausgehend von erhaltenen Endstoffen der Formel I, worin B für Cb und mindestens eines der Symbole $AA^1$, $AA^2$ und $AA^3$ für Ac oder Cb steht, zu solchen Endstoffen gelangt, worin $AA^1$, $AA^2$ und $AA^3$ alle Wasserstoff bedeuten. Die Ammonolyse wird ebenfalls in an sich bekannter, konventioneller Weise durchgeführt. Ueblicherweise wird eine Aufschwemmung, oder vorzugsweise eine Lösung des umzusetzenden O-Acylats in einem inerten organischen Lösungsmittel, vornehmlich in Abwesenheit von Wasser, mit trockenem Ammoniakgas (vorzugsweise mit einem Ueberschuss davon) bei Temperaturen zwischen etwa 0° bis etwa 45°C, insbesondere im Bereich der Raumtemperatur, und beim atmosphärischen Druck behandelt. Geeignete Lösungsmittel sind beispielsweise halogenierte Niederalkane (wie Dichlormethan, Chloroform und 1,2-Dichlorethan), Niederalkanole (wie insbesondere Methanol, Ethanol, Isopropylalkohol und tert-Butylalkohol), aliphatische und heterocyclische Ether (wie Diethylether und 1,2-Dimethoxyethan bzw. Tetrahydrofuran und Dioxan), aliphatische Amide vom Typ Acetamid und N,N-Dimethylformamid, und Acetonitril und ähnliche Niederalkylcyanide, sowie zweckmässige Gemische davon. Besonders gut bewährt sich diese nachträgliche Umwandlung in Fällen, in welchen die oben beschriebenen Acylierungsmethoden keine einheitlichen Resultate ergeben, indem sie z.B. bei einer erwünschten N-Acylierung teilweise auch die freien Hydroxylgruppen im Ausgangsstoff angreifen, oder aber bei totaler Acylierung nur unvollständig acylierte Produkte liefern.

Auch die erfindungsgemässe nachträgliche Abspaltung der Schutzgruppe X erfolgt in der allgemein bekannten Weise, wobei spezifische Bedingungen für einzelne Strukturtypen in der einschlägigen Literatur (siehe z.B. Houben-Weyl, loc. cit.) bis ins Detail beschrieben sind. Die Acidolyse (einschliesslich saurer Hydrolyse) wird z.B. mit Trifluoressigsäure, Fluorwasserstoff, Bromwasserstoff und Chlorwasserstoff, gegebenenfalls in Anwesenheit von Wasser, wie mit Salzsäure, und bei säureempfindlichen Schutzgruppen auch mit einer niederaliphatischen Carbonsäure, wie Ameisensäure und/oder Essigsäure, gegebenenfalls in Anwesenheit von Wasser und/oder von einem polyhalogenierten Niederalkanol oder Niederalkanon, wie 1,1,1,3,3,3-Hexafluorpropan-2-ol oder Hexafluoraceton, durchgeführt. Die neutral abspaltbaren Gruppen, insbesondere solche, die Benzylreste enthalten, werden vorzugsweise hydrogenolytisch, z.B. durch Hydrierung unter Palladium-Katalyse, entfernt. Die β-Silylethoxycarbonylgruppen werden vorzugsweise mit Fluoridionen-abgebenden Mitteln, z.B. mit Fluoriden quaternärer organischer Basen, wie Tetraethylammoniumfluorid oder Tetrabutylammoniumfluorid, in neutralen organischen Lösungsmitteln abgespalten.

Je nach Arbeitsweise erhält man die Endstoffe der Formel I mit unsubstituierter endständiger Aminogruppe in Form von Basen oder Säureadditionssalzen. Aus den Säureadditionssalzen können in an sich bekannter Weise die Basen gewonnen werden. Von letzteren wiederum lassen sich durch Umsetzen mit Säuren, z.B. mit solchen, die die oben genannten Salze bilden, therapeutisch anwendbare Säureadditionssalze gewinnen.

Die wichtigsten Ausgangsstoffe der Formel II, wie insbesondere Desferrioxamin B und seine Säureadditionssalze, sind bekannt oder, sofern sie unbekannt sind, wie einige Derivate Desferrioxamins B mit geschützter Aminogruppe, insbesondere solche, worin in der Formel II das Symbol $X_0$ eine von Resten $Cb_0$ unterschiedliche Bedeutung hat, kann man in an sich bekannter Weise unter Anwendung allgemeiner Methoden, wie sie z.B. insbesondere in der Peptidchemie geläufig sind, erhalten. Dabei wird z.B. vornehmlich so vorgegangen, dass man im unsubstituierten Desferrioxamin B die freie Aminogruppe durch die gewünschte Schutzgruppe blockiert, indem man beispielsweise Desferrioxamin B (als freie Base

oder Säureadditionssalz) mit einem Aminoschutzgruppe-einführenden Reaktionsmittel behandelt, vorzugsweise unter Bedingungen, die zur selektiven Funktionalisierung der Aminogruppe unter Erhaltung freier Hydroxylgruppen führen. Dies erfolgt vornehmlich beispielsweise durch Umsetzen mit einem Reagens der Formel $X_oY$, worin Y Halogen, insbesondere Chlor, ist und $X_o$ für die einzuführende Schutzgruppe, wie insbesondere Trityl oder (gegebenenfalls substituiertes) Phenylsulfenyl steht, unter bekannten allgemeinen Bedingungen.

Die zur Veresterung vorzugsweise zu verwendenden Chlorameisensäureester, sofern sie nicht bereits bekannt sind, können in an sich bekannter, konventioneller Weise, z.B. durch Behandeln eines entsprechenden Alkohols bzw. Glykolmonoethers mit einer äquivalenten Menge Phosgen, gegebenenfalls in Anwesenheit eines nicht-acylierbaren Amins (wie eines oben genannten) erhalten werden. Auch die Methoden zur Herstellung anderer reaktiver Acylierungsmitteln, z.B. aktiver Ester, sind allgemein bekannt.

Beim Verfahren der vorliegenden Erfindung werden vorzugsweise solche Ausgangsstoffe verwendet, welche zu den eingangs als besonders wertvoll geschilderten Verbindungen führen.

Die Erfindung betrifft auch diejenigen Ausführungsformen des Verfahrens, bei denen ein Ausgangsstoff in Form eines Derivats davon, zum Beispiel eines Salzes, verwendet wird.

Die vorliegende Erfindung betrifft ebenfalls pharmazeutische Zusammensetzungen, die als Wirkstoff eine der neuen pharmakologisch wirksamen Verbindungen der Formel I, insbesondere eine der oben für diese Anwendung hervorgehobenen, enthalten. Besonders bevorzugt sind Präparate und Zusammensetzungen zur enteralen, wie insbesondere oralen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab.

Die pharmazeutischen Zusammensetzungen enthalten von etwa 5% bis etwa 95% des Wirkstoffs, wobei einzeldosierte Applikationsformen vorzugsweise von etwa 20% bis etwa 90% und nichteinzeldosierte Applikationsformen vorzugsweise etwa 5% bis etwa 20% Wirkstoff aufweisen; pharmazeutische Präparate in Dosiseinheitsform, wie Dragées, Tabletten oder Kapseln, enthalten von etwa 0,05 g bis etwa 2,0 g, vorzugsweise von etwa 0,1 g bis etwa 1,0 g des Wirkstoffs.

Die Pharmazeutischen Zusammensetzungen der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragées-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol.

Dragées-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere, oral anwendbare pharmazeutische Zusammensetzungen sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 5% bis 20%, vorzugsweise ca. 10% oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Die Verbindungen der Formel I können z.B. in einem Verfahren zur Behandlung von Krankheiten verwendet werden, bei denen, wie oben beschrieben worden ist, ein Ueberschuss von Eisen(III) oder Aluminium im Körper vorhanden ist, und wobei man eine prophylaktisch oder therapeutisch wirksame Menge einer Verbindung der Formel I, vorzugsweise peroral, verabreicht. Dabei verwendet man in erster

Linie die obengenannten pharmazeutischen Zusammensetzungen, wobei man einem Warmblüter von etwa 70 kg Gewicht eine tägliche Dosis von etwa 0,5 g bis etwa 15 g, vorzugsweise von etwa 1,5 g bis etwa 7,5 g einer Verbindung der vorliegenden Erfindung verabreicht.

Die nachfolgenden Beispiele illustrieren die vorliegende Erfindung.

Dabei beschreiben die Beispiele 1 und 6—8 keine Endstoffe der Formel I, sondern Ausgangsstoffe oder sinngemäss übertragbare Verfahrensweisen zur Herstellung der Endstoffe. Temperaturen werden in Celsiusgraden angegeben.

Beispiel 1

N-tert-Butoxycarbonyl-desferrioxamin B:

Eine Suspension von 59,7 g (100 mMol) Desferrioxamin B-Hydrochlorid in 900 ml eines Gemisches (v/v) von Dioxan-Wasser (2:1) wird mit 10,6 g (100 mMol) wasserfreiem Natriumcarbonat und 26,6 ml (123 mMol) Di-tert-butyl-dicarbonat versetzt und 1,75 Stunden bei 35—40° gerührt. Die entstandene klare blassgelbe Lösung wird bei etwa 50° (Badtemperatur) im Vakuum zur Trockne eingeengt; der kristalline Rückstand wird bei 50° in 150 ml Methanol suspendiert, abgekühlt, mit 1 Liter Diethylether versetzt und 0,5 Stunden bei 22° stehen gelassen. Die Kristalle werden abgenutscht und mit 500 ml Diethylether gewaschen, womit sie 59,9 g der Titelverbindung, Smp. 133—137° ergeben; aus der eingeengten Mutterlauge können in analoger Weise mit Methanol-Diethylether noch weitere 8,7 g Kristalle eines weniger reinen Produkts gewonnen werden.

In analoger Weise kann mit gleichem Resultat auch Desferrioxamin B-Methansulfonat verarbeitet werden.

Beispiel 2

N-tert-Butoxycarbonyl-O,O′,O′′-tripivsloyl-desferrioxamin B:

Eine Lösung von 9,9 g (15 mMol) N-tert-Butoxy-desferrioxamin B (siehe Beispiel 1) in 25 ml absolutem N,N-Dimethylformamid und 8,9 ml (60 mMol) 1,8-Diazabicyclo[5.4.0]undec-7-en wird bei 0° mit 9,2 ml (75 mMol) Pivalinsäurechlorid versetzt und ohne weitere Kühlung 4 Stunden bei Raumtemperatur gerührt. Die Reaktionslösung wird unter Rühren in ein Gemisch von je 300 ml 0,3-molarer wässriger Dikaliumhydrogenphosphat-Lösung und Ethylacetat gegossen. Nach Sättigen mit Natriumchlorid trennt man die Phasen und extrahiert die untere noch zweimal mit Ethylacetat nach. Die organischen Auszüge werden mit 0,3-molarer Dikaliumhydrogenphosphat-Lösung und gesättigter Natriumchlorid-Lösung gewaschen, über Natriumsulfat getrocknet und im Vakuum zum harzartigen Rohprodukt eingedampft. Chromatographie an der 30-fachen Menge Kieselgel gibt in den mit Methylenchlorid-Methanol (95:5) eluierten Fraktionen die gewünschte Titelverbindung.

Dünnschichtchromatographie (Kieselgel):

$R_f$: 0,40—0,45; Toluol-Aceton (4:6)

Beispiel 3

N-tert-Butoxycarbonyl-O,O′,O′′-triacetyl-desferrioxamin B

In analoger Weise wie im Beispiel 2 und unter Anwendung derselben Mengen der Komponenten wird N-tert-Butoxycarbonyl-desferrioxamin B (siehe Beispiel 1) mit 7,5 mMol Acetylchlorid umgesetzt und zum N-tert-Butoxycarbonyl-O,O′,O′′-triacetyl-desferrioxamin B verarbeitet.

Dünnschichtchromatographie (Kieselgel):

$R_f$: 0,40—0,45; Toluol-Aceton (4:6).

Beispiel 4

N-tert-Butoxycarbonyl-O,O′,O′′-tribenzoyl-desferrioxamin B

In analoger Weise wie im Beispiel 2 und unter Anwendung derselben Mengen der Komponenten wird N-tert-Butoxycarbonyl-desferrioxamin B (siehe Beispiel 1) mit 7,5 mMol Benzoylchlorid umgesetzt und zum N-tert-Butoxycarbonyl-O,O′,O′′-tribenzoyl-desferrioxamin B umwandelt.

Dünnschichtchromatographie (Kieselgel):

$R_f$: 0,40—0,45; Toluol-Aceton (4:6).

Beispiel 5

N-tert-Butoxycarbonyl-O,O′,O′′-trioctanoyl-desferrioxamin B

Eine intensiv gerührte Suspension von 13,22 g (20 mMol) N-tertButoxycarbonyl-desferrioxamin B (siehe Beispiel 1) in 100 ml Dichlormethan, 500 ml Tetrahydrofuran, 250 ml Dioxan und 11,2 ml (80 mMol) Triethylamin wird bei 23°C während 25 Minuten mit einer Lösung von 12,1 ml (70,7 mMol) Octanoylchlorid in 20 ml Dichlormethan tropfenweise behandelt und zusätzlich bei Raumtemperatur weitere 6 Stunden gerührt. Die Lösungsmittel werden im Wasserstrahlvakuum entfernt, der Rückstand mit 50 ml Wasser vermischt und mit Ethylacetat extrahiert. Die organischen Auszüge werden mit Natriumsulfat getrocknet, vom Lösungsmittel im Vakuum befreit, in Dichlormethan gelöst und über Kieselgel chromatographiert. Durch Eluieren mit Gemischen von Dichlormethan-Isopropylalkohol (95:5) und Abdestillieren der Lösungsmittel wird das gewünschte N-tert-Butoxycarbonyl-O,O′,O′′-trioctanoyl-desferrioxamin B als

dickes Oel erhalten, welches allmählich zu Kristallen, Smp. 47-50°C, erstarrt.

Dünnschichtchromatographie (Kieselgel):

$R_f$: 0,27; Chloroform-Aceton (70:30).

Beispiel 6

N,O,O',O'' -Tetraethoxycarbonyl-desferrioxamin B

Eine Suspension von 98,4 g (0,15 Mol) Desferrioxamin B-Methansulfonat in 1500 ml Acetonitril und 3000 ml Methylenchlorid wird mit 209 ml (1,5 Mol) Triethylamin versetzt und unter Rühren bei 10°C mit einer Lösung von 144 ml (1,5 Mol) Chlorameisensäure-ethylester in 200 ml Methylenchlorid während 30 Minuten tropfenweise behandelt. Das Reaktionsgemisch wird weitere 3 Stunden bei Raumtemperatur gerührt und im Wasserstrahlvakuum von leichtflüchtigen Komponenten befreit. Der Rückstand wird mit 1 Liter Phosphatpuffer von pH 8,0 verrührt, auf pH 7,4 gestellt und in Methylenchlorid aufgenommen. Durch Abdestillieren des organischen Lösungsmittels resultiert als einheitliches Produkt die gewünschte Titelverbindung in Form eines gelben Oels.

Dünnschichtchromatographie (Kieselgel):

$R_f$: 0,26; Methylenchlorid-Isopropylalkohol (9:1)
0,82; Chloroform-Methanol (4:1)

Beispiel 7

N-Ethoxycarbonyl-desferrioxamin B

In eine Lösung von 127,0 g (0,15 Mol) rohem N,O,O',O''-Tetraethoxycarbonyl-desferrioxamin B (siehe Beispiel 6) in 2 Liter Methylenchlorid wird bei Raumtemperatur während 5 Stunden trockenes Ammoniakgas eingeleitet. Das Reaktionsgemisch wird eingeengt und der h kristalline Rückstand aus Ethylacetat kristallisiert. Die er altene Titelverbindung, Smp. 157—158°C, ist einheitlich gemäss Dünnschichtchrsmatographie:

[Kieselgel; $R_f$: 0,20 in Methylenchlorid-Isopropylalkohol (9:1) und 0,65 in Chloroform-Methanol (4:1)].

Beispiel 8

N-Ethoxycarbonyl-O,O',O''-trioctanoyl-desferrioxamin B

Eine Suspension von 12,6 g (20 mMol) N-Ethoxycarbonyl-desferrioxamin B in 500 ml Acetonitril und 300 ml Methylenchlorid wird mit 11 ml (80 mMol) Triethylamin versetzt und unter Rühren bei 22°C mit einer Lösung von 12 ml (72 mMol) Octanoylchlorid in 20 ml Methylenchlorid tropfenweise während 30 Minuten behandelt. Das Reaktionsgemisch wird bei Temperaturen zwischen 30°C und Raumtemperatur weitere 5 Stunden gerührt und durch Destillation im Wasserstrahlvakuum von leichtflüchtigen Anteilen befreit, der Rückstand mit Wasser verrührt, mit Eisessig auf pH 3,0 gestellt und in Ethylacetat aufgenommen. Die organische Phase ergibt nach Einengen das rohe Produkt als gelbrötliches Oel. Dieses wird in Methylenchlorid gelöst und über Kieselgelsäule chromatographiert; die mit einem Gemisch von Methylenchlorid-Isopropylalkohol (95:5) eluierten Fraktionen ergeben nach Abdestillieren der Lösungsmittel die gewünschte Titelverbindung als fast farbloses Oel, welches gemäss Dünnschichtchromatograpie einheitlich ist.

Dünnschichtchromatographie (Kieselgel):

$R_f$ = 0,21; Methylenchlorid-Isopropylalkohol (9:1);
0,80; Chloroform-Methanol (4:1)

Beispiel 9

N-tert-Butoxycarbonyl-O,O',O''-tri-(2-methoxyethoxycarbonyl)-desferrioxamin B [N-tert-Butoxycarbonyl-O,O',O''-tri-(3oxa-butoxycarbonyl)-desferrioxamin B].

Zu einer Suspension von 662 mg (1,0 mMol) N-tert-Butoxycarbonyl-desferrioxamin B (siehe Beispiel 1) in 25 ml Tetrahydrofuran, 6 ml Methylenchlorid und 0,56 ml (4,0 mMol) Triethylamin wird während 2 Minuten bei 23°C eine Lösung von 693 mg (5,0 mMol) 2-Methoxyethyl-chloroformiat in 1,0 ml Methylenchlorid zugegeben und das Gemisch 45 Minuten bei Raumtemperatur gerührt. Flüchtige Anteile der Reaktionsmischung werden im Vakuum abdestilliert, der feste Rückstand in 20 ml Wasser aufgenommen und mit Ethylacetat extrahiert. Durch Abdestillieren des Lösungsmittels aus den organischen Auszügen resultiert ein Rohprodukt, welches in Methylenchlorid-Lösung auf eine Kieselgelsäule aufgetragen wird. Elution mit einem Gemisch von Methylenchlorid-Isopropylalkohol (92:8) ergibt Fraktionen, die nach Abdestillieren der Lösungsmittel die gewünschte Titelverbindung als farbloses, gemäss Dünnschichtchromatographie einheitliches Oel liefern.

Dünnschichtchromatographie (Kieselgel):

$R_f$ = 0,05 in Chloroform-Aceton (70:30),
0,4 in Methylenchlorid-Isopropylalkohol (90:10) und
0,85 in Methylenchlorid-Methanol (80:20)

Das als Reagens verwendete 2-Methoxyethyl-chloroformiat kann folgendermassen erhalten werden:

In 520 ml einer 20%-igen Lösung von Phosgen in Toluol (entsprechend 98,9 g, d.h. 1,0 Mol, Phosgen) wird während 45 Minuten unter Kühlung auf 5—10°C und mit Rühren ein Gemisch von 39,6 ml (0,5 Mol) 2-Methoxyethenol und 73,5 ml (0,53 Mol) Triethylamin zugetropft und das Gemisch eine weitere Stunde bei

Zimmertemperatur gerührt. Das überschüssige Phosgen wird durch Durchleiten eines Stickstoffstroms bei 30-40°C entfernt und mit einer wässrigen 4N-Natriumhydroxid-Lösung aufgefangen. Das Reaktionsgemisch wird im Vakuum fraktioniert; das gewünschte 2-Methoxyethyl-chloroformiat resultiert als eine bei 59—61°C/16 Torr (/2133 Pa) siedende farblose Flüssigkeit in einer Ausbeute von 20,9 g (30% der Theorie).

Beispiel 10

N-tert-Butoxycarbonyl-O,O',O''-tri-[2-(2-methoxyethoxy)-ethoxycarbonyl]-desferrioxamin B [N-tert-Butoxycarbonyl-O,O',O''-tri-(3,6-dioxa-heptyloxycarbonyl)-desferrioxamin B]

In analoger Weise wie im Beispiel 9 werden gleiche Mengen der Desferrioxamin B-Verbindung und Hilfsstoffe mit einer Lösung von 922 mg (5,05 mMol) 2-(2-Methoxyethoxy)-ethyl-chloroformiat in 3,5 ml Toluol versetzt, 4 Stunden bei Raumtemperatur gerührt und verarbeitet. Das Rohprodukt wird in gleicher Weise wie im Beispiel 9 chromatographisch gereinigt und ergibt die Titelverbindung als dünnschichtchromatographisch einheitliches, farbloses Oel.

Dünnschichtchromatographie (Kieselgel):

$R_f$ 0,03 in Chloroform-Aceton (70:30)

0,23 in Methylenchlorid-Isopropylalkohol (90:10) und

0,77 in Methylenchlorid-Methanol 80:20).

Das als Reagens verwendete 2-(2-Methoxyethoxy)-ethyl-chloroformiat kann durch das im Anhang zum Beispiel 9 beschriebene allgemeine Verfahren, jedoch mit 60 ml (0,5 Mol) 2-(2-Methoxyethoxy)-ethanol als Alkohol-Komponente, hergestellt werden.

Beispiel 11

O,O',O''-Tri-(3-oxabutoxycarbonyl)-desferrioxamin B-Hydrochlorid

Eine Lösung von 7,64 g (7,9 mMol) N-tert-Butoxycarbonyl-O,O',O''-tri-(3-oxabutoxcycarbonyl)-desferrioxamin B (siehe Beispiel 9) in 80 ml Methylenchlorid wird mit 80 ml einer wasserfreien 0,5N-Lösung von Chlorwasserstoff in Methylenchlorid versetz un 2,5 Stunden bei 22° gerührt. Das Reaktionsgemisch wird mit 80 ml Ethylacetat verdünnt und im Vakuum weitgehend eingeengt. Der Rückstand wird mit Gemischen von Ether-Petrolether digeriert und an Kieselgel chromatographiert; Elution mit einem Gemisch von Methylenchlorid-Methanol (80:20) ergibt die Titelverbindung in praktisch reinem Zustand.

Dünnschichtchromatographie (Kieselgel):

$R_f$: 0,52-0,55 in Chloroform-Methanol (9:l).

Beissiel 12

O,O',O''-Tri-(3,6-dioxa-heptyloxycarbonyl)-desferrioxamin B-Trifluoracetat

Zu einer Lösung von 2,2 g (2,0 mMol) N-tert-Butoxycarbonyl-O,O',O''-tri-(3,6-dioxa-heptyloxy-carbonyl)-desferrioxamin B (siehe Beispiel 10) in 10 ml Toluol und 1,5 ml (3,8 mMol) Anisol wird bei 2°C während 3 Minuten 6,0 ml (80,8 mMol) Trifluoressigsäure zugetropft und das Reaktionsgemisch eine Stunde bei einer Temperatur unterhalb 10°C gerührt. Das Lösungsmittel wird entfernt und der Rückstand im Hochvakuum bis zur Gewichtskonstanz bei 45—50°C getrocknet, wodurch die Titelverbindung resultiert.

Dünnschichtchromatographie (Kieselgel):

$R_f$: 0,02; Chloroform-Aceton (70:30).

Beispiel 13

Herstellung von 1000 Kapseln mit 260 mg des Wirkstoffes (d.h. des Produkts gemäss einem der Beispiele 2—5 und 9—12) pro Kapsel

Zusammensetzung

| | |
|---|---|
| Wirkstoff | 260 g |
| Talk | 36 g |
| Weizenstärke | 24 g |
| Magnesiumstearat | 16 g |
| Laktose | 4 g |
| | 340 g |

Zubereitung

Die pulverförmigen Substanzen werden durch ein Sieb mit einer Maschenweite von 0,6 mm getrieben und gründlich gemischt. Mit je 340 mg dieser Mischung werden mit einer Kapselfüllmaschine Gelatine-Kapseln bereitet.

Beispiel 14

Herstellung von 1000 Kapseln enthaltend 105 mg des Wirkstoffes (d.h. des Produkts gemäss einem der Beispiele 2—5 und 9—12) pro Kapsel

Zusammensetzung

| | |
|---|---|
| Wirkstoff | 105 g |
| Ethylcellulose | 3 g |
| Stearinsäure | 3 g |
| | 111 g |

Zubereitung

Die Ethylcellulose und die Stearinsäure werden in 120 ml Methylenchlorid gelöst, mit dem Wirkstoff versetzt, und die Masse wird durch ein Sieb von 0,6 mm Maschenweite bei einer Temperatur von ca. 40° geschlagen, wobei das Methylenchlorid verdampft. 111 mg des erhaltenen Granulates werden in Gelatine-Kapseln zu 0,5 ml mit Hilfe einer Kapsel-Abfüllmaschine abgefüllt.

**Patentansprüche für die Vertragsstaaten: BE CH DE FR GB IT LI LU NL SE**

1. Ein Acylat des Desferrioxamins B der Formel

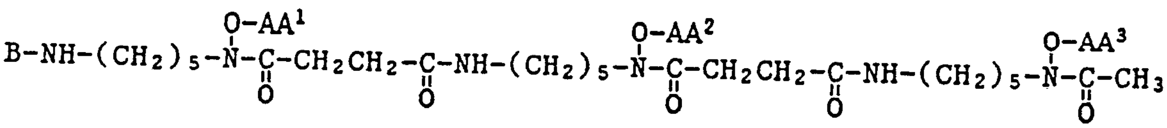

(I)

mit der folgenden Bedeutung der Symbole:

$AA^1$, $AA^2$ und $AA^3$, jedes unabhängig, ist ein als Ac bezeichneter Acylrest einer Carbonsäure mit 1—24 C-Atomen oder ein als Cb bezeichneter veresterter Oxycarbonylrest (Acylrest eines Kohlensäuremonoesters) mit insgesamt 2—25 C-Atomen, und

B hat eine von Ethoxycarbonyl und Benzyloxycarbonyl unterschiedliche Bedeutung von Cb oder, falls mindestens eines der Symbole $AA^1$, $AA^2$ und $AA^3$ für Cb steht, kann auch Wasserstoff oder eine Aminoschutzgruppe, ausgewählt aus mono- oder bicyclischen α-Arylniederalkoxycarbonylresten, die von einem Benzyloxycarbonylrest verschieden sind, aus sekundären oder tertiären Alkoxycarbonyl- oder Cycloalkoxycarbonylresten, sowie aus β-(Tri-hydrocarbyl-silyl)-ethoxycarbonylresten und Allyloxycarbonyl, sein,

sowie Salze davon, falls dieses Acylat salzbildende Eigenschaften besitzt.

2. Eine Verbindung gemäss Anspruch 1 mit den folgenden Bedeutungen der Symbole in Formel I: $AA^1$, $AA^2$ und $AA^3$, jeweils alle zusammen, sind Acylreste, welche, jeder unabhängig von den anderen, eine Bedeutung von Ac haben, oder Oxycarbonylreste, welche, jeder unabhängig von anderen, eine Bedeutung von Cb haben, oder ein Salz einer solchen Verbindung mit salzbildenden Eigenschaften.

3. Eine Verbindung gemäss Anspruch 1, worin in der Formel I $AA^1$, $AA^2$ und $AA^3$ jeweils alle für identisches Ac oder identisches Cb stehen, oder ein Salz einer solchen Verbindung mit salzbildenden Eigenschaften.

4. Eine Verbindung gemäss Anspruch 1, worin in Formel (I) B Wasserstoff, eine der im Anspruch 1 genannten Aminoschutzgruppen oder einen Oxycarbonylrest der Teilformel $R—CH_2—O—CO—$ darstellt, in welchem R Wasserstoff oder ein von Methyl und einem Phenylrest verschiedenes, gegebenenfalls substituiertes Hydrocarbyl mit 1—23 C-Atomen ist, und $AA^1$, $AA^2$ und $AA^3$ unabhängig je einen solchen, wie oben definierten Oxycarbonylrest darstellen, oder ein pharmazeutisch verwendbares Salz einer solchen Verbindung mit salzbildenden Eigenschaften.

5. Eine Verbindung gemäss Anspruch 4, worin in Formel (I) B Wasserstoff und $AA^1$, $AA^2$ und $AA^3$ je ein Alkoxycarbonyl, dessen Kohlenstoffkette durch 1, 2 oder mehrere nicht-benachbarte O-Atome unterbrochen sein kann, oder ein ($C_6$—$C_{11}$-Alkenyl)-oxycarbonyl darstellen, oder ein pharmazeutisch anwendbares Säureadditionssalz davon.

6. Eine Verbindung gemäss Anspruch 4, worin in Formel (I) B, $AA^1$, $AA^2$ und $AA^3$ je dasselbe von Ethoxycarbonyl verschiedene Alkoxycarbonyl, dessen Kohlenstoffkette durch 1, 2 oder mehrere nichtbenachbarte O-Atome unterbrochen sein kann, oder ein ($C_6$—$C_{11}$-Alkenyl)-oxycarbonyl darstellen, oder ein pharmazeutisch anwendbares Säureadditionssalz davon.

7. Eine Verbindung gemäss Anspruch 4, worin in Formel (I) B eine der im Anspruch 1 genannten und vom Oxycarbonylrest der Formel $R_o—O—CO—$, worin $R_o$ ein neutral und/oder acidolytisch abspaltbares Hydrocarbyl ist, unterschiedlichen Aminoschutzgruppen und $AA^1$, $AA^2$ und $AA^3$ je ein Alkoxycarbonyl, dessen Kohlenstoffkette durch 1, 2 oder mehrere nicht-benachbarte O-Atome unterbrochen sein kann, oder

ein ($C_6$—$C_{11}$-Alkenyl)-oxycarbonyl darstellen.

8. Eine Verbindung gemäss Anspruch 1 oder 4, worin in Formel (I) B einen von Ethoxycarbonyl und Benzyloxycarbonyl verschiedenen, als Aminoschutzgruppe anwendbaren Oxycarbonylrest der Formel $R_o$—O—CO—, worin $R_o$ ein neutral und/oder acidolytisch abspaltbares Hydrocarbyl ist, und $AA^1$, $AA^2$ und $AA^3$ je ein Alkoxycarbonyl, dessen Kohlenstoffkette durch 1, 2 oder mehrere nicht-benachbarte O-Atome unterbrochen sein kann, oder ein ($C_6$—$C_{11}$-Alkenyl)-oxycarbonyl darstellen.

9. Eine Verbindung gemäss Anspruch 1, worin in Formel (I) B einen von Benzyloxycarbonyl unterschiedlichen, als Aminoschutzgruppe anwendbaren Oxycarbonylrest der Formel $R_o$—O—CO—, worin $R_o$ ein neutral und/oder acidolytisch abspaltbares Hydrocarbyl ist, darstellt.

10. Eine Verbindung gemäss einem der Ansprüche 7—9, worin $R_o$ tert-Butyl, Allyl, 2-(Trimethylsilyl)-ethyl oder ein durch Nitro, Chlor, Methyl oder Methoxy substituiertes Benzyl darstellt.

11. N,O,O',O''-Tetra-(alkoxycarbonyl)-desferrioxamin B nach Anspruch 1 mit Ausnahme von N,O,O',O''-Tetra-(ethoxycarbonyl)-desferrioxamin B.

12. N,O,O',O''-Tetra-[(3-oxa-, 3,6-dioxa- oder 3,6,9-trioxa)-alkoxycarbonyl]-desferrioxamin B nach Anspruch 1.

13. O,O',O''-Tri-(alkoxycarbonyl)-desferrioxamin B oder ein pharmazeutisch anwendbares Säureadditionssalz davon nach Anspruch 1.

14. O,O',O''-Tri-[(3-oxa-, 3,6-dioxa- oder 3,6,9-trioxa)-alkoxycarbonyl]-desferrioxamin B oder ein pharmazeutisch anwendbares Säureadditionssalz davon nach Anspruch 1.

15. O,O',O''-Tri-(3-oxa-butoxy-carbonyl)-desferrioxamin B oder ein pharmazeutisch anwendbares Säureadditionssalz davon nach Anspruch 1.

16. O,O',O''-Tri-(3,6-dioxa-heptyloxycarbonyl)-desferrioxamin B oder ein pharmazeutisch anwendbares Säureadditionssalz davon nach Anspruch 1.

17. N-tert.Butoxycarbonyl-O,O',O''-tri-(2-methoxy-ethoxy-carbonyl)-desferrioxamin B nach Anspruch 1.

18. N-tert. Butoxycarbonyl-O,O',O''-tri-[2-(2-methoxy-ethoxy)-ethoxy-carbonyl)-desferrioxamin B nach Anspruch 1.

19. N-tert. Butoxycarbonyl-O,O',O''-tri-octanoyl-desferrioxamin B nach Anspruch 1.

20. Eine Verbindung der Formel I gemäss einem der Ansprüche 1—19 oder ein pharmazeutisch verwendbares Salz einer solchen Verbindung mit salzbildenden Eigenschaften zur Anwendung für die therapeutische Behandlung des menschlichen oder tierischen Körpers.

21. Eine Verbindung gemäss einem der Ansprüche 1—19 zur Anwendung für Bildung von Eisen(III)- und/oder Aluminium-Komplexen in lebender Zelle.

22. Verfahren zur Herstellung einer Verbindung der Formel I nach Anspruch 1 oder eines Salzes davon, falls dieses Acylat salzbildende Eigenschaften besitzt dadurch gekennzeichnet dass man

a) eine Verbindung der Formel

$$B_o-NH-(CH_2)_5-\underset{}{N}(O-A^1)-C(=O)-CH_2CH_2-C(=O)-NH-(CH_2)_5-N(O-A^2)-C(=O)-CH_2CH_2-C(=O)-NH-(CH_2)_5-N(O-A^3)-C(=O)-CH_3$$

(II)

worin $A^1$, $A^2$ und $A^3$, jedes unabhängig, Wasserstoff ist oder eine der in Anspruch 1 definierten Bedeutungen von Ac hat und $B_o$ Wasserstoff ist oder, falls mindestens eines der Symbole $A^1$, $A^2$ und $A^3$ Wasserstoff ist, auch eine von einer Oxycarbonylgruppe unterschiedliche Aminoschutzgruppe $X_o$ sein kann, mit einem vom, im Anspruch 1 definierten, Rest Cb abgeleiteten reaktionsfähigen Derivat eines Kohlensäuremonoesters acyliert, oder

b) eine Verbindung der Formel I, worin B für einen von Ethoxycarbonyl oder Benzyloxycarbonyl unterschiedlichen Rest Cb und $AA^1$, $AA^2$ und $AA^3$ für Wasserstoff stehen, zu einer Verbindung der Formel I, worin B für einen von Ethoxycarbonyl oder Benzyloxycarbonyl unterschiedlichen Rest Cb steht und $AA^1$, $AA^2$ und $AA^3$ unabhängig voneinander die Bedeutung von Ac und/oder Cb haben, acyliert, oder

c) eine Verbindung der Formel I, worin mindestens eines der Symbole $AA^1$, $AA^2$ und $AA^3$ eine der Bedeutungen von Cb hat und B für eine Aminoschutzgruppe X steht, durch Abspalten dieser Aminoschutzgruppe in eine entsprechende Verbindung der Formel I, worin B Wasserstoff ist, oder in ein Salz davon umwandelt, und/oder,

gewünschtenfalls, eine freie Verbindung der Formel I mit salzbildenden Eigenschaften in ein Salz umwandelt und/oder eine Verbindung der Formel I aus einem solchen Salz freisetzt.

23. Verfahren gemäss Anspruch 22 [Verfahren a) oder b)], dadurch gekennzeichnet, dass man mit einem Ueberschuss an einem Chlorameisensäureester CbCl, worin Cb die im Anspruch 1 genannten Bedeutungen hat, in Anwesenheit eines stark basischen säurebindenden Mittels sowohl die Aminogruppe wie such freie Hydroxylgruppen acyliert.

24. Verfahren gemäss Anspruch 22 [Verfahren a)], dadurch gekennzeichnet, dass man mit einer äquivalenten Menge eines symmetrischen Kohlensäureanhydrids der Formel Cb—O—Cb, worin Cb die im Anspruch 1 genannten Bedeutungen hat, oder eines entsprechenden Aktivesters in Anwesenheit eines

basischen säurebindenden Mittels selektiv die Aminogruppe acyliert.

25. Verfahren gemäss einem der Ansprüche 22—26, dadurch gekennzeichnet dass man eine in den Ansprüchen 2—19 charakterisierte Verbindung der Formel I herstellt.

26. Pharmazeutische Zusammensetzungen, enthaltend als Wirkstoff eine Verbindung der Formel I gemäss einem der Ansprüche 1—19 oder ein pharmazeutisch verwendbares Salz einer solchen Verbindung mit salzbildenden Eigenschaften zusammen mit pharmazeutischen Trägermaterial.

27. Pharmazeutische Zusammensetzungen gemäss Anspruch 26 zur oralen Verabreichung.

28. Pharmazeutische Zusammensetzungen gemäss Anspruch 27, die sich aufgrund des pharmazeutischen Trägermaterials nicht zur parenteralen, wohl aber zur oralen Verabreichung eignen.

29. Verwendung einer Verbindung gemäss einem der Ansprüche 1—19 zur Herstellung eines Arzneimittels für die Behandlung von krankhaften Zuständen in Warmblütern, einschliesslich Menschen, welche mit einem Ueberschuss an Eisen(III) oder Aluminium im Körper zusammenhängen.

30. Verwendung einer Verbindung gemäss einem der Ansprüche 1—19 zur Herstellung eines Arzneimittels für die Behandlung von krankhaften Zuständen in Warmblütern, einschliesslich Menschen, welche durch Eisen(III)-abhängige pathogene Organismen verursacht sind.

31. Anwendung einer Verbindung gemäss einem der Ansprüche 1—19 als Zwischenprodukt zur Herstellung therapeutisch wirksamer Derivate des Desferrioxamins B.

**Patentansprüche für den Vertragsstaat: AT**

1. Verfahren zur Herstellung eines Acylats des Desferioxamin B der Formel

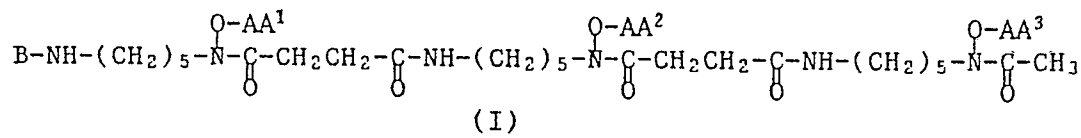

(I)

mit der folgenden Bedeutung der Symbole:

$AA^1$, $AA^2$ und $AA^3$, jedes unabhängig, ist, ein als Ac bezeichneter Acylrest einer Carbonsäure mit 1—24 C-Atomen, oder ein als Cb bezeichneter veresterter Oxycarbonylrest (Acylrest eines Kohlensäuremonoesters) mit insgesamt 2—25 C-Atomen, und

B hat eine von Ethoxycarbonyl und Benzyloxycarbonyl unterschiedliche Bedeutung von Cb oder, falls mindestens eines der Symbole $AA^1$, $AA^2$ und $AA^3$ für Cb steht, kann auch Wasserstoff oder eine Aminoschutzgruppe, ausgewählt aus mono- oder bicyclischen α-Arylniederalkoxycarbonylresten, die von einem Benzyloxycarbonylrest verschieden sind, aus sekundären oder tertiären Akoxycarbonyl- oder Cycloalkoxycarbonylresten sowie aus β-(Tri-hydrocarbyl-silyl)-ethoxycarbonylresten und Allyloxycarbonyl sein oder eines

Salzes davon, falls dieses Acylat salzbildende Eigenschaften besitzt, dadurch gekennzeichnet, dass man

a) eine Verbindung der Formel

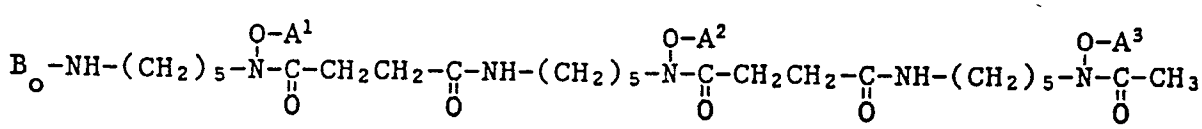

(II)

worin $A^1$, $A^2$ und $A^3$, jedes unabhängig, Wasserstoff ist oder eine der in Anspruch 1 definierten Bedeutungen von Ac hat und $B_o$ Wasserstoff ist oder, falls mindestens eines der Symbole $A^1$, $A^2$ und $A^3$ Wasserstoff ist, auch eine von einer Oxycarbonylgruppe unterschiedliche Aminoschutzgruppe $X_o$ sein kann, mit einem vom, im Anspruch 1 definierten, Rest Cb abgeleiteten reaktionsfähigen Derivat eines Kohlensäuremonoesters acyliert, oder

b) eine Verbindung der Formel I, worin B für einen von Ethoxycarbonyl oder Benzyloxycarbonyl unterschiedlichen Rest Cb und $AA^1$, $AA^2$ und $AA^3$ für Wasserstoff stehen, zu einer Verbindung der Formel I, worin B für einen von Ethoxycarbonyl oder Benzyloxycarbonyl unterschiedlichen Rest Cb steht und $AA^1$, $AA^2$ und $AA^3$ unabhängig voneinander die Bedeutung von Ac und/oder Cb haben, acyliert, oder

c) eine Verbindung der Formel I, worin mindestens eines der Symbole $AA^1$, $AA^2$ und $AA^3$ eine der Bedeutungen von Cb hat und B für eine Aminoschutzgruppe X steht, durch Abspalten dieser Aminoschutzgruppe in eine entsprechende Verbindung der Formel I, worin B Wasserstoff ist, oder in ein Salz davon umwandelt, und/oder,

gewünschtenfalls, eine freie Verbindung der Formel I mit salzbildenden Eigenschaften in ein Salz umwandelt und/oder eine Verbindung der Formel I aus einem solchen Salz freisetzt.

2. Verfahren gemäss Anspruch 1 [Verfahren a) oder b)], dadurch gekennzeichnet, dass man mit einem Ueberschuss an einem Chlorameisensäureester CbCl, worin Cb die im Anspruch 1 genannten Bedeutungen hat, in Anwesenheit eines stark basischen säurebindenden Mittels sowohl die Aminogruppe wie auch freie Hydroxylgruppen acyliert.

3. Verfahren gemäss Anspruch 1 [Verfahren a)], dadurch gekennzeichnet, dass man mit einer äquivalenten Menge eines symmetrischen Kohlensäureanhydrids der Formel Cb—O—Cb, worin Cb die im Anspruch 1 genannten Bedeutungen hat, oder eines entsprechenden Aktivesters in Anwesenheit eines basischen säurebindenden Mittels selektiv die Aminogruppe acyliert.

4. Verfahren nach Anspruch 1, dadurch gekennzeichnet, dass man die Ausgangsstoffe so wählt, dass man eine Verbindung der Formel I mit den folgenden Bedeutungen der Symbole in Formel I: $AA^1$, $AA^2$ und $AA^3$, jeweils alle zusammen, sind Acylreste, welche, jeder unabhängig von den anderen, eine Bedeutung von Ac haben, oder Oxycarbonylreste, welche, jeder unabhängig von anderen, eine Bedeutung von Cb haben, oder ein Salz einer solchen Verbindung mit salzbildenden Eigenschaften herstellt.

5. Verfahren nach Anspruch 1, dadurch gekennzeichnet, dass man die Ausgangsstoffe so wählt, dass man eine Verbindung der Formel I, worin $AA^1$, $AA^2$ und $AA^3$ jeweils alle für identisches Ac oder identisches Cb stehen, oder ein Salz einer solchen Verbindung mit salzbildenden Eigenschaften herstellt.

6. Verfahren nach Anspruch 1 dadurch gekennzeichnet dass man die Ausgangsstoffe so wählt, dass man eine Verbindung der Formel I, worin B Wasserstoff, eine der im Anspruch 1 genannten Aminoschutzgruppen genannten oder einen Oxycarbonylrest der Teilformel $R—CH_2—O—CO—$ darstellt, in welchem R Wasserstoff oder ein von Methyl und einem Phenylrest verschiedenes, gegebenenfalls substituiertes Hydrocarbyl mit 1—23 C-Atomen ist, und $AA^1$, $AA^2$ und $AA^3$ unabhängig je einen solchen, wie oben definierten Oxycarbonylrest darstellen, oder ein pharmazeutisch verwendbares Salz einer solchen Verbindung mit salzbildenden Eigenschaften herstellt.

7. Verfahren nach Anspruch 6, dadurch gekennzeichnet, dass man die Ausgangsstoffe so wählt, dass man eine Verbindung der Formel I, worin B Wasserstoff und $AA^1$, $AA^2$ und $AA^3$ je ein Alkoxycarbonyl, dessen Kohlenstoffkette durch 1, 2 oder mehrere nicht-benachbarte O-Atome unterbrochen sein kann, oder ein ($C_6$—$C_{11}$-Alkenyl)-oxycarbonyl darstellen, oder ein pharmazeutisch anwendbares Säureadditionssalz davon herstellt.

8. Verfahren nach Anspruch 6, dadurch gekennzeichnet, dass man die Ausgangsstoffe so wählt, dass man eine Verbindung der Formel I, worin B, $AA^1$, $AA^2$ und $AA^3$ je dasselbe von Ethoxycarbonyl verschiedene Alkoxycarbonyl, dessen Kohlenstoffkette durch 1, 2 oder mehrere nicht-benachbarte O-Atome unterbrochen sein kann, oder ein ($C_6$—$C_{11}$-Alkenyl)-oxycarbonyl darstellen, oder ein pharmazeutisch anwendbares Säureadditionssalz davon herstellt.

9. Verfahren nach Anspruch 6, dadurch gekennzeichnet, dass man die Ausgangsstoffe so wählt, dass man eine Verbindung der Formel I herstellt, worin B eine der im Anspruch 1 genannten und vom Oxycarbonylrest der Formel $R_o$—O—CO—, worin $R_o$ ein neutral und/oder acidolytisch abspaltbares Hydrocarbyl ist, unterschiedlichen Aminoschutzgruppen und $AA^1$, $AA^2$ und $AA^3$ je ein Alkoxycarbonyl, dessen Kohlenstoffkette durch 1, 2 oder mehrere nicht-benachbarte O-Atome unterbrochen sein kann, oder ein ($C_6$—$C_{11}$—Alkenyl)-oxycarbonyl darstellen.

10. Verfahren nach Anspruch 1 oder 6, dadurch gekennzeichnet, dass man die Ausgangsstoffe so wählt, dass man eine Verbindung der Formel I herstellt, worin B einen von Ethoxycarbonyl und Benzyloxycarbonyl verschiedenen, als Aminoschutzgruppe anwendbaren Oxycarbonylrest der Formel $R_o$—O—CO—, worin $R_o$ ein neutral und/oder acidolytisch abspaltbares Hydrocarbyl ist, und $AA^1$, $AA^2$ und $AA^3$ je ein Alkoxycarbonyl, dessen Kohlenstoffkette durch 1, 2 oder mehrere nicht-benachbarte O-Atome unterbrochen sein kann, oder ein ($C_6$—$C_{11}$-Alkenyl)-oxycarbonyl darstellen.

11. Verfahren nach Anspruch 1, dadurch gekennzeichnet, dass man die Ausgangsstoffe so wählt, dass man eine Verbindung der Formel I herstellt, worin B einen von Benzyloxycarbonyl unterschiedlichen, als Aminoschutzgruppe anwendbaren Oxycarbonylrest der Formel $R_o$—O—CO—, worin $R_o$ ein neutral und/oder acidolytisch abspaltbares Hydrocarbyl ist, darstellt.

12. Verfahren nach einem der Ansprüche 9—11, dadurch gekennzeichnet, dass man die Ausgangsstoffe so wählt, dass man eine Verbindung der Formel I herstellt, worin $R_o$ tert-Butyl, Allyl, 2-(Trimethylsilyl)-ethyl oder ein durch Nitro, Chlor, Methyl oder Methoxy substituiertes Benzyl darstellt.

13. Verfahren nach Anspruch 1, dadurch gekennzeichnet, dass man die Ausgangsstoffe so wählt, dass man ein N,O,O',O''-Tetra-(ethoxycarbonyl)-desferrioxamin B mit Ausnahme von N,O,O',O''-Tetra(ethoxycarbonyl)-desferrioxamin B herstellt.

14. Verfahren nach Anspruch 1, dadurch gekennzeichnet, dass man die Ausgangsstoffe so wählt, dass man ein N,O,O',O''-Tetra-[(3-oxa-, 3,6-dioxa- oder 3,6,9-trioxa)-alkoxycarbonyl]-desferrioxamin B herstellt.

15. Verfahren nach Anspruch 1, dadurch gekennzeichnet, dass man die Ausgangsstoffe so wählt, dass man ein O,O',O''-Tri-(alkoxycarbonyl)-desferrioxamin B oder ein pharmazeutisch anwendbares Säureadditionssalz davon herstellt.

16. Verfahren nach Anspruch 1, dadurch gekennzeichnet, dass man die Ausgangsstoffe so wählt, dass man ein O,O',O''-Tri-[(3-oxa-, 3,6-dioxa- oder 3,6,9-trioxa)-alkoxycarbonyl]-desferrioxamin B oder ein pharmazeutisch anwendbares Säureadditionssalz davon herstellt.

17. Verfahren nach Anspruch 1, dadurch gekennzeichnet, dass man die Ausgangsstoffe so wählt, dass man O,O',O''-Tri-(3-oxa-butoxycarbonyl)-desferrioxamin B oder ein pharmazeutisch anwendbares Säureadditionssalz davon herstellt.

18. Verfahren nach Anspruch 1, dadurch gekennzeichnet, dass man die Ausgangsstoffe so wählt, dass man O,O',O''-Tri-(3,6-dioxa-heptyloxycarbonyl)-desferrioxamin B oder ein pharmazeutisch anwendbares Säureadditionssalz davon herstellt.

19. Verfahren nach Anspruch 1, dadurch gekennzeichnet, dass man die Ausgangsstoffe so wählt, dass man N-tert-Butoxycarbonyl-O,O',O''-tri-(2-methoxy-ethoxy-carbonyl)-desferrioxamin B herstellt.

20. Verfahren nach Anspruch 1, dadurch gekennzeichnet, dass man die Ausgangsstoffe so wählt, dass man N-tert. Butoxycarbonyl-O,O',O''-tri-[2-(2-methoxy-ethoxy)-ethoxy-carbonyl]-desferrioxamin B herstellt.

21. Verfahren nach Anspruch 1, dadurch gekennzeichnet, dass man die Ausgangsstoffe so wählt, dass man N-tert. Butoxycarbonyl-O,O',O''-tri-octanoyl-desferrioxamin B herstellt.

**Revendications pour les Etats contractants: BE CH DE FR GB IT LI LU NL SE**

1. Un acylate de la desferrioxamine B de formule

```
                   O-AA¹                            O-AA²                            O-AA³
                   |                                |                                |
B-NH-(CH2)5-N-C-CH2CH2-C-NH-(CH2)5-N-C-CH2CH2-C-NH-(CH2)5-N-C-CH3
              ||        ||                ||        ||                ||
              O         O                 O         O                 O
```

(I)

dans laquelle

$AA^1$, $AA^2$ et $AA^3$ représentent chacun, indépendamment les uns des autres, le radical acyle Ac d'un acide carboxylique en C1—C24, ou un groupe oxycarbonyle estérifié (radical acyle d'un monoester carbonique) Cb contenant au total 2 à 25 atomes de carbone, et

B a l'une des significations de Cb à l'exception des groupes éthoxycarbonyle et benzyloxycarbonyle ou bien, lorsque l'un au moins des symboles $AA^1$, $AA^2$ et $AA^3$ a l'une des significations de Cb, B représente l'hydrogène ou un groupe protecteur du groupe amino choisi parmi les groupes alpha-aryl-(alcoxy inférieur)-carbonyle mono- ou bicycliques autres que le groupe benzyloxycarbonyle, les groupes alcoxycarbonyle ou cycloalcoxycarbonyle secondaires ou tertiaires, ou les groupes bêta-(tri-hydrocarbyl-silyl)-éthoxycarbonyle et allyloxycarbonyle,

et les sels de cet acylate lorsqu'il est salifiable.

2. Un composé selon la revendication 1, pour lequel les symboles de la formule I ont les significations suivantes: $AA^1$, $AA^2$ et $AA^3$ sont tous des radicaux acyle qui, indépendamment les uns des autres, ont l'une des significations de Ac, ou des groupes oxycarbonyle qui, indépendamment les uns des autres, ont l'une des significations de Cb, ou un sel d'un tel composé lorsqu'il est salifiable.

3. Un composé selon la revendication 1, pour lequel, dans la formule I, $AA^1$, $AA^2$ et $AA^3$ représentent tous des groupes Ac identiques ou Cb identiques, ou un sel d'un tel composé lorsqu'il est salifiable.

4. Un composé selon la revendication 1, pour lequel, dans la formule I, B représente l'hydrogène, l'un des groupes protecteurs du groupe amino mentionnés dans la revendication 1 ou un groupe oxycarbonyle de formule partielle $R—CH_2—O—CO—$ dans laquelle R représente l'hydrogène ou un groupe hydrocarboné en C1—C23 éventuellement substitué autre qu'un groupe méthyle ou phényle, et $AA^1$, $AA^2$ et $AA^3$ représentent chacun, indépendamment les uns des autres, un groupe oxycarbonyle tel que défini ci-dessus, ou un sel acceptable pour l'usage pharmaceutique d'un tel composé lorsqu'il est salifiable.

5. Un composé selon la revendication 4, pour lequel, dans la formule I, B représente l'hydrogène et $AA^1$, $AA^2$ et $AA^3$ représentent chacun, indépendamment les uns des autres, un groupe alcoxycarbonyle dont la chaîne carbonée peut être interrompue par un, deux ou plusieurs atomes d'oxygène non voisins, ou un groupe (alcényle en C6—C11)-oxycarbonyle, ou un sel acceptable pour l'usage pharmaceutique d'un tel composé.

6. Un composé selon la revendication 4, pour lequel, dans la formule I, B, $AA^1$, $AA^2$ et $AA^3$ représentent chacun le même groupe alcoxycarbonyle autre qu'un groupe éthoxycarbonyle, dont la chaîne carbonée peut être interrompue par un, deux ou plusieurs atomes d'oxygène non voisins, ou un groupe (alcényle en C6—C11)-oxycarbonyle, ou un sel acceptable pour l'usage pharmaceutique d'un tel composé.

7. Un composé selon la revendication 4, pour lequel, dans la formule I, B représente l'un des groupes protecteurs du groupe amino mentionnés dans la revendication 1 et autre qu'un groupe oxycarbonyle de formule $R_o—O—CO—$ dans laquelle $R_o$ représente un groupe hydrocarboné éliminable en milieu neutre et/ou par acidolyse, et $AA^1$, $AA^2$ et $AA^3$ représentent chacun un groupe alcoxycarbonyle dont la chaîne carbonée peut être interrompue par un, deux ou plusieurs atomes d'oxygène non voisins, ou un groupe (alcényle en C6—C11)-oxycarbonyle.

8. Un composé selon la revendication 1 ou 4, pour lequel, dans la formule I, B représente un groupe oxycarbonyle utilisable comme groupe-protecteur du groupe amino et autre qu'un groupe éthoxycarbonyle ou benzyloxycarbonyle, et répondant à la formule $R_o—O—CO—$ dans laquelle $R_o$ représente un groupe hydrocarboné éliminable en milieu neutre et/ou par acidolyse, et $AA^1$, $AA^2$ et $AA^3$ représentent chacun un groupe alcoxycarbonyle dont la chaîne carbonée peut être interrompue par un, deux ou plusieurs atomes d'oxygène non voisins, ou un groupe (alcényle en C6—C11)-oxycarbonyle.

9. Un composé selon la revendication 1 pour lequel, dans la formule I, B représente un groupe oxycarbonyle utilisable comme groupe protecteur du groupe amino autre qu'un groupe

benzyloxycarbonyle et répondant à la formule $R_o$—O—CO— dans laquelle $R_o$ est un groupe hydrocarboné éliminable en milieu neutre et/ou par acidolyse.

10. Un composé selon l'une des revendications 7 à 9, pour lequel $R_o$ représente un groupe tert-butyle, allyle, 2-(triméthylsilyl)-éthyle ou un groupe benzyle substitué par des groupes nitro, le chlore, des groupes méthyle ou méthoxy.

11. Une N,O,O',O''-tétra-(alcoxycarbonyl)-desferrioxamine B selon la revendication 1, à l'exception de la N,O,O',O''-tétra-(éthoxycarbonyl)-desferrioxamine B.

12. Une N,O,O',O''-tétra-[(3-oxa-, 3,6-dioxa- ou 3,6,9-trioxa)-alcoxycarbonyl]-desferrioxamine B selon la revendication 1.

13. Une O,O',O''-tri-(alcoxycarbonyl)-desferrioxamine B ou un sel d'un tel composé acceptable pour l'usage pharmaceutique selon la revendication 1.

14. Une O,O',O''-tri-[(3-oxa-, 3,6-dioxa- ou 3,6,9-trioxa)-alcoxycarbonyl]-desferrioxamine B ou un sel acceptable pour l'usage pharmaceutique d'un tel composé, selon la revendication 1.

15 La O,O',O''-tri-(3-oxa-butoxy-carbonyl)-desferrioxamine B ou un sel acceptable pour l'usage pharmaceutique de ce composé selon la revendication 1.

16. La O,O',O''-tri-(3,6-dioxa-heptyloxycarbonyl)-desferrioxamine B ou un sel de ce composé acceptable pour l'usage pharmaceutique selon la revendication 1.

17. La N-tert-butoxycarbonyl-O,O',O''-tri-(2-méthoxy-éthoxy-carbonyl)-desferrioxamine B selon la revendication 1.

18. La N-tert-butoxycarbonyl-O,O',O''-tri-[2(2-méthoxy-éthoxy)-éthoxy-carbonyl]-desferrioxamine B selon la revendication 1.

19. La N-tert-butoxycarbonyl-O,O',O''-trioctanoyl-desferrioxamine B selon la revendication 1.

20. Un composé de formule I selon l'une des revendications 1 à 19, ou un sel acceptable pour l'usage pharmaceutique d'un tel composé lorsqu'il est salifiable, pour l'utilisation pour le traitement thérapeutique de l'organisme humain ou animal.

21. Un composé selon l'une des revendications 1 à 19, pour l'utilisation pour la formation de complexes de fer-III et/ou d'aluminium dans la cellule vivante.

22. Procédé de préparation d'un composé de formule I selon la revendication 1 ou d'un sel d'un tel composé lorsque cet acylate est salifiable, caractérisé en ce que:

a) on acyle un composé de formule

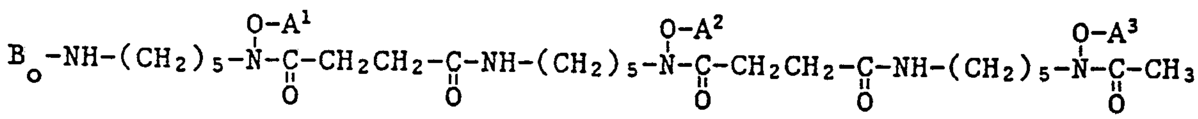

(II)

dans laquelle $A^1$, $A^2$ et $A^3$, indépendamment les uns des autres, représentent l'hydrogène ou ont l'une des significations de Ac indiquées dans la revendication 1 et $B_o$ représente l'hydrogène ou bien encore, lorsque l'un au moins des symboles $A^1$, $A^2$ et $A^3$ représente l'hydrogène, un groupe protecteur du groupe amino autre qu'un groupe oxycarbonyle, $X_o$, par un dérivé réactif d'un monoester carbonique dérivant du groupe Cb défini dans la revendication 1, ou bien

b) on acyle un composé de formule I dans laquelle B représente un groupe Cb autre qu'un groupe éthoxycarbonyle ou benzyloxycarbonyle et $AA^1$, $AA^2$ et $AA^3$ représentent l'hydrogène, en un composé de formule I dans laquelle B représente un groupe Cb autre qu'un groupe éthoxycarbonyle ou benzyloxycarbonyle et $AA^1$, $AA^2$ et $AA^3$ ont chacun, indépendamment les uns des autres, l'une des significations de Ac et/ou de Cb, ou bien,

c) on convertit un composé de formule I dans laquelle l'un au moins des symboles $AA^1$, $AA^2$ et $AA^3$ a l'une des significations de Cb et B représente un groupe protecteur du groupe amino X, par élimination de ce groupe protecteur du groupe amino, en un composé correspondant de formule I dans laquelle B représente l'hydrogène, ou en un sel d'un tel composé, et/ou,

si on le désire, on convertit un composé libre de formule I, étant salifiable, en un sel, et/ou on libère un composé de formule I à partir d'un tel sel.

23. Procédé selon la revendication 22 [procédé a) ou b)], caractérisé en ce que l'on acyle à l'aide d'un excès d'un ester chloroformique CbCl dans lequel Cb a les significations indiquées dans la revendication 1, en présence d'un accepteur d'acide fortement basique, de sorte qu'il y a acylation du groupe amino et également des groupes hydroxy libres.

24. Procédé selon la revendication 22 [procédé a)], caractérisé en ce que l'on acyle à l'aide d'une quantité équivalente d'un anhydride carbonique symétrique de formule Cb—O—Cb dans laquelle Cb a les significations indiquées dans la revendication 1, ou d'un ester actif correspondant, en présence d'un accepteur d'acide basique, de sorte qu'il y a acylation sélective du groupe amino.

25. Procédé selon l'une quelconque des revendications 22 à 26, caractérisé en ce que l'on prépare les composés de formule I caractérisés dans les revendications 2 à 19.

26. Compositions pharmaceutiques contenant en tant que substance active un composé de formule I

selon l'une des revendications 1 à 19, ou un sel acceptable pour l'usage pharmaceutique d'un tel composé salifiable, avec un véhicule pharmaceutique.

27. Compositions pharmaceutiques selon la revendication 26, pour l'administration orale.

28. Compositions pharmaceutiques selon la revendication 27, qui en raison de la nature du véhicule pharmaceutique, ne conviennent pas pour l'administration parentérale mais conviennent pour l'administration orale.

29. Utilisation d'un composé selon l'une des revendications 1 à 19 pour la préparation d'un médicament pour le traitement des états pathologiques des individus à sang chaud, y compris les humains, en relation avec un excès de fer-III ou d'aluminium dans l'organisme.

30. Utilisation d'un composé selon l'une des revendications 1 à 19 pour la préparation d'un médicament pour le traitement des états pathologiques des individus à sang chaud, y compris les humains, causés par des microorganismes pathogènes en relation avec le fer-III.

31. Utilisation d'un composé selon l'une des revendications 1 à 19 en tant que produit intermédiaire de la préparation de dérivés de la desferrioxamine B possédant une activité thérapeutique.

**Revendications pour l'Etat contractant: AT**

1. Procédé de préparation d'un acylate de la desferrioxamine B, de formule

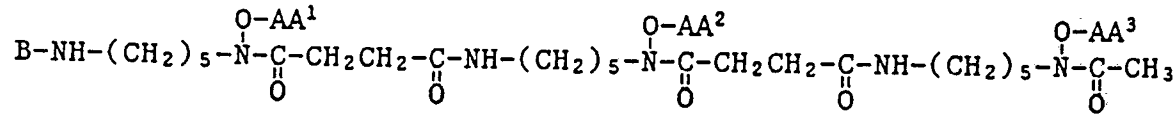

(I)

dans laquelle

$AA^1$, $AA^2$ et $AA^3$ représentent chacun, indépendamment les uns des autres, un radical acyle Ac d'un acide carboxylique en C1—C24 ou un groupe oxycarbonyle estérifié (radical acyle d'un monoester carbonique) Cb contenant au total 2 à 25 atomes de carbone, et

B a l'une des significations de Cb, autre que le groupe éthoxycarbonyle ou benzyloxycarbonyle, ou bien encore, lorsque l'un au moins des symboles $AA^1$, $AA^2$ et $AA^3$ a l'une des significations de Cb, il représente l'hydrogène ou un groupe protecteur du groupe amino choisi parmi les groupes α-aryl-(alcoxy inférieur)-carbonyle mono- ou bi-cycliques autres que le groupe benzyloxycarbonyle, les groupes alcoxycarbonyle ou cycloalcoxycarbonyle secondaires ou rertiaires, ou les groupes β-(tri-hydrocarbyl-silyl)-éthoxycarbonyle et allyloxycarbonyle,

ou d'un sel d'un tel acylate lorsqu'il est salifiable, caractérisé en ce que:

a) on acyle un composé de formule

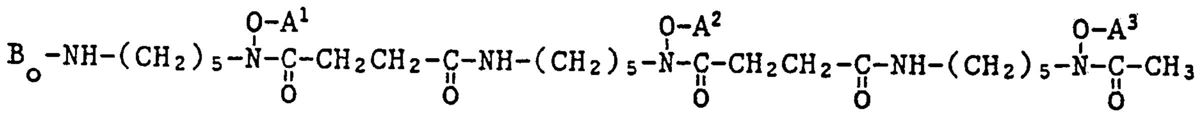

(II)

dans laquelle $A^1$, $A^2$ et $A^3$ indépendamment les uns des autres, représentent l'hydrogène ou ont l'une des significations de Ac indiquées dans la revendication 1, et $B_o$ représente l'hydrogène ou bien encore, lorsque l'un au moins des symboles $A^1$, $A^2$ et $A^3$ représente l'hydrogène, un groupe protecteur du groupe amino $X_o$ autre qu'un groupe oxycarbonyle, par un dérivé réactif d'un monoester carbonique dérivant du groupe Cb défini dans la revendication 1 ou bien,

b) on acyle un composé de formule I dans laquelle B représente un groupe Cb autre qu'un groupe éthoxycarbonyle ou benzyloxycarbonyle, et $AA^1$, $AA^2$ et $AA^3$ représentent l'hydrogène, en un composé de formule I dans laquelle B représente un groupe Cb autre qu'un groupe éthoxycarbonyle ou benzyloxycarbonyle, et $AA^1$, $AA^2$ et $AA^3$ ont chacun, indépendamment les uns des autres, l'une des significations de Ac et/ou Cb, ou bien

c) On convertit un composé de formule I dans laquelle l'un au moins des symboles $AA^1$, $AA^2$ et $AA^3$ a l'une des significations de Cb et B représente un groupe protecteur du groupe amino X, par élimination de ce groupe protecteur du groupe amino, en un composé correspondant de formule I dans laquelle B représente l'hydrogène, ou en un sel d'un tel composé, et/ou,

si on le désire, on convertit un composé libre de formule I, salifiable, en un sel, et/ou on libère un composé de formule I à partir d'un tel sel.

2. Procédé selon la revendication 1, [procédé a) ou b)], caractérisé en ce que l'on acyle à l'aide d'un excès d'un ester chloroformique CbCl pour lequel Cb a l'une des significations indiquées dans la revendication 1, en présence d'un accepteur d'acide fortement basique, de sorte qu'il y a acylation du groupe amino et également des groupes hydroxy libres.

3. Procédé selon la revendication 1 [procédé a)], caractérisé en ce que l'on acyle à l'aide d'une quantité

équivalent d'un anhydride carbonique symétrique de formule Cb—O—Cb dans laquelle Cb a l'une des significations indiquées dans la revendication 1, ou d'un ester actif correspondant, en présence d'un accepteur d'acide basique, de sorte qu'il y a acylation sélective du groupe amino.

4. Procédé selon la revendication 1, caractérisé en ce que l'on choisit les composants de départ de manière à obtenir un composé de formule I dans laquelle $AA^1$, $AA^2$ et $AA^3$ représentent tous des radicaux acyle ayant, indépendamment les uns des autres, l'une des significations de Ac, ou des groupes oxycarbonyle ayant, indépendamment les uns des autres, l'une des significations de Cb, ou un sel d'un tel composé salifiable.

5. Procédé selon la revendication 1, caractérisé en ce que l'on choisit les composants de départ de manière à obtenir un composé de formule I dans laquelle $AA^1$, $AA^2$ et $AA^3$ représentent tous des groupes Ac identiques ou Cb identiques, ou un sel d'un tel composé lorsqu'il est salifiable.

6. Procédé selon la revendication 1, caractérisé en ce que l'on choisit les composants de départ de manière à obtenir un composé de formule I dans laquelle B représente l'hydrogène, l'un des groupes protecteurs du groupe amino mentionnés dans la revendication 1, ou un groupe oxycarbonyle de formule partielle $R—CH_2—O—CO—$ dans laquelle R représente l'hydrogène ou un groupe hydrocarboné éventuellement substitué en C1—C23 autre qu'un groupe méthyle ou phényle, et $AA^1$, $AA^2$ et $AA^3$ représentent chacun, indépendamment les uns des autres, un groupe oxycarbonyle tel que défini ci-dessus, ou un sel acceptable pour l'usage pharmaceutique d'un tel composé lorsqu'il est salifiable.

7. Procédé selon la revendication 6, caractérisé en ce que l'on choisit les composants de départ de manière à obtenir un composé de formule I dans laquelle B représente l'hydrogène et $AA^1$, $AA^2$ et $AA^3$ représentent chacun un groupe alcoxycarbonyle dont la chaîne carbonée peut être interrompue par un, deux ou plusieurs atomes d'oxygène non voisins, ou un groupe (alcényle en C6—C11)-oxycarbonyle, ou un sel acceptable pour l'usage pharmaceutique d'un tel composé.

8. Procédé selon la revendication 6, caractérisé en ce que l'on choisit les composants de départ de manière à obtenir un composé de formule I dans laquelle B, $AA^1$, $AA^2$ et $AA^3$ représentent chacun le même groupe alcoxycarbonyle, autre qu'un groupe éthoxycarbonyle, dont la chaîne carbonée peut être interrompue par un, deux ou plusieurs atomes d'oxygène non voisins, ou un groupe (alcényle en C6—C11)-oxycarbonyle, ou un sel d'un tel composé formé par addition avec un acide acceptable pour l'usage pharmaceutique.

9. Procédé selon la revendication 6, caractérisé en ce que l'on choisit les composants de départ de manière à obtenir un composé de formule I dans laquelle B représente l'un des groupes protecteurs du groupe amino mentionnés dans la revendication 1 et autre qu'un groupe oxycarbonyle de formule $R_o—O—CO—$ dans laquelle $R_o$ représente un groupe hydrocarboné éliminable en milieu neutre et/ou par acidolyse, et $AA^1$, $AA^2$ et $AA^3$ représentent chacun un groupe alcoxycarbonyle dont la chaîne carbonée peut être interrompue par un, deux ou plusieurs atomes d'oxygène non vqisins, ou un groupe (alcényle en C6—C11)-oxycarbonyle.

10. Procédé selon la revendication 1 ou 6, caractérisé en ce que l'on choisit les composants de départ de manière à obtenir un composé de formule I dans laquelle B représente un groupe oxycarbonyle de formule $R_o—O—CO—$ dans laquelle $R_o$ représente un groupe hydrocarboné éliminable en milieu neutre et/ou par acidolyse, utilisable en tant que groupe protecteur du groupe amino, autre qu'un groupe éthoxycarbonyle ou benzyloxycarbonyle, et $AA^1$, $AA^2$ et $AA^3$ représentent chacun un groupe alcoxycarbonyle dont la chaîne carbonée peut être interrompue par un deux ou plusieurs atomes d'oxygène non voisins, ou un groupe (alcényle en C6—C11)-oxycarbonyle.

11. Procédé selon la revendication 1, caractérisé en ce que l'on choisit les composants de départ de manière à obtenir un composé de formule I dans laquelle B représente un groupe oxycarbonyle de formule $R_o—O—CO—$ dans laquelle $R_o$ représente un groupe hydrocarboné éliminable en milieu neutre et/ou par acidolyse, utilisable en tant que groupe protecteur du groupe amino, et autre qu'un groupe benzyloxycarbonyle.

12. Procédé selon l'une des revendications 9 à 11, caractérisé en ce que l'on choisit les composants de départ de manière à obtenir un composé de formule I dans laquelle $R_o$ représente un groupe tert-butyle, allyle, 2-(triméthylsilyl)-éthyle ou un groupe benzyle substitué par des groupes nitro, le chlore, des groupes méthyle ou méthoxy.

13. Procédé selon la revendication 1, caractérisé en ce que l'on choisit les composants de départ de manière à obtenir une N,O,O',O''-tétra-(éthoxycarbonyl)-desferrioxamine B, à l'exception de la N,O,O',O''-tétra(éthoxycarbonyl)-desferrioxamine B.

14. Procédé selon la revendication 1, caractérisé en ce que l'on choisit les composants de départ de manière à obtenir une N,O,O',O''-tétra-[(3-oxa-, 3,6-dioxa- ou 3,6,9-trioxa)-alcoxycarbonyl]-desferrioxamine B.

15. Procédé selon la revendication 1, caractérisé en ce que l'on choisit les composants de départ de manière à obtenir une O,O',O''-tri-(alcoxycarbonyl)-desferrioxamine B ou un sel d'un tel composé formé par addition avec un acide acceptable pour l'usage pharmaceutique.

16. Procédé selon la revendication 1, caractérisé en ce que l'on choisit les composants de départ de manière à obtenir une O,O',O''-tri-[(3-oxa-, 3,6-dioxa- ou 3,6,9-trioxa)-alcoxycarbonyl]-desferrioxamine B ou un sel d'un tel composé formé par addition avec un acide acceptable pour l'usage pharmaceutique.

17. Procédé selon la revendication 1, caractérisé en ce que l'on choisit les composants de départ de

manière à obtenir la O,O',O''-tri-(3-oxa-butoxycarbonyl)-desferrioxamine B ou un sel de ce composé formé par addition avec un acide acceptable pour l'usage pharmaceutique.

18. Procédé selon la revendication 1, caractérisé en ce que l'on choisit les composants de départ de manière à obtenir la O,O',O''-tri-(3,6-dioxa-heptyloxycarbonyl)-desferrioxamine B ou un sel de ce composé formé par addition avec un acide acceptable pour l'usage pharmaceutique.

19. Procédé selon la revendication 1, caractérisé en ce que l'on choisit les composants de départ de manière à obtenir la N-tert-butoxycarbonyl-O,O',O''-tri-(2-méthoxy-éthoxy-carbonyl)-desferrioxamine B.

20. Procédé selon la revendication 1, caractérisé en ce que l'on choisit les composants de départ de manière à obtenir la N-tert-butoxycarbonyl-O,O',O''-tri-[2-(2-méthoxy-éthoxy)-éthoxy-carbonyl]-desferrioxamine B.

21. Procédé selon la revendication 1, caractérisé en ce que l'on choisit les composants de départ de manière à obtenir la N-tert-butoxycarbonyl-O,O',O''-trioctanoyl-desferrioxamine B.

**Claims for the Contracting States: BE CH DE FR GB IT LI LU NL SE**

1. An acylate of desferrioxamine B of the formula

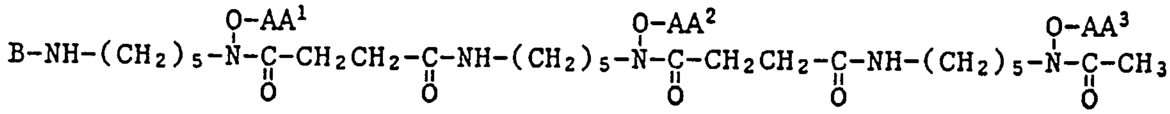

(I)

the symbols having the following meanings:

each of $AA^1$, $AA^2$ and $AA^3$, independently of the others, is an acyl radical, referred to as Ac, of a carboxylic acid having from 1 to 24 carbon atoms, or an esterified oxycarbonyl radical referred to as Cb (acyl radical of a carbonic acid monoester) having a total of from 2 to 25 carbon atoms, and

B has one of the meanings of Cb other than ethoxycarbonyl or benzyloxycarbonyl, or, if at least one of the symbols $AA^1$, $AA^2$ and $AA^3$ represents Cb, it may also be hydrogen or an amino-protecting group selected from mono, or bicyclic α-aryl-lower alkoxycarbonyl radicals other than a benzyloxycarbonyl radical, from secondary or tertiary alkoxycarbonyl or cycloalkoxycarbonyl radicalsand from β-(trihydrocarbyl-silyl)-ethoxycarbonyl radicals and allyloxycarbonyl,

and salts thereof if this acylate has salt-forming properties.

2. A compound according to claim 1, the symbols in formula I having the following meanings: all of $AA^1$, $AA^2$ and $AA^3$ are acyl radicals, each of which, independently of the others, has one of the meanings of Ac, or all are oxycarbonyl radicals, each of which, independently of the others, has one of the meanings of Cb, or a salt of such a compound having salt-forming properties.

3. A compound according to claim 1 in which, in the formula I, $AA^1$, $AA^2$ and $AA^3$ all represent identical Ac or all represent identical Cb, or a salt of such a compound having salt-forming properties.

4. A compound according to claim 1 in which, in formula (I), B represents hydrogen, one of the amino-protecting groups mentioned in claim 1 or an oxycarbonyl radical of the partial formula $R—CH_2—O—CO—$ in which R is hydrogen or an unsubstituted or substituted hydrocarbyl radical having from 1 to 23 carbon atoms other than methyl or a phenyl radical, and $AA^1$, $AA^2$ and $AA^3$, independently of one another, each represents an oxycarbonyl radical of the type defined above, or a pharmaceutically acceptable salt of such a compound having salt-forming properties.

5. A compound according to claim 4 in which, in formula (I), B represents hydrogen and $AA^1$, $AA^2$ and $AA^3$ each represents an alkoxycarbonyl radical whose carbon chain may be interrupted by 1, 2 or more non-vicinal oxygen atoms, or a ($C_6$—$C_{11}$-alkenyl)-oxycarbonyl radical, or a pharmaceutically acceptable acid addition salt thereof.

6. A compound according to claim 4, in which, in formula (I), B, $AA^1$, $AA^2$ and $AA^3$ each represents the same alkoxycarbonyl radical, other than ethoxycarbonyl, whose carbon chain may be interrupted by 1, 2 or more non-vicinal oxygen atoms, or a ($C_6$—$C_{11}$-alkenyl)-oxycarbonyl radical, or a pharmaceutically acceptable acid addition salt thereof.

7. A compound according to claim 4 in which, in formula (I), B represents one of the amino-protecting groups mentioned in claim 1 other than the oxycarbonyl radical of the formula $R_o—O—CO—$ in which $R_o$ is a hydrocarbyl radical that can be removed under neutral conditions and/or by acidolysis, and $AA^1$, $AA^2$ and $AA^3$ each represents an alkoxycarbonyl radical whose carbon chain may be interrupted by 1, 2 or more non-vicinal oxygen atoms, or a ($C_6$—$C_{11}$-alkenyl)-oxycarbonyl radical.

8. A compound according to claim 1 or 4, in which, in formula (I), B is an oxycarbonyl radical, other than ethoxycarbonyl or benzyloxycarbonyl, that can be used as an amino-protecting group and has the formula $R_o—O—CO—$ in which $R_o$ is a hydrocarbyl radical that can be removed under neutral conditions and/or by acidolysis, and $AA^1$, $AA^2$ and $AA^3$ each represents an alkoxycarbonyl radical whose carbon chain may be interrupted by 1, 2 or more non-vicinal oxygen atoms or a ($C_6$—$C_{11}$-alkenyl)-oxycarbonyl radical.

9. A compound according to claim 1 in which, in formula (I), B represents an oxycarbonyl radical, other than benzyloxycarbonyl, that can be used as an amino-protecting group and has the formula $R_o—O—CO—$

in which $R_o$ is a hydrocarbyl radical that can be removed under neutral conditions and/or by acidolysis.

10. A compound according to any of claims 7 to 9, in which $R_o$ represents tert.-butyl, allyl, 2-(trimethylsilyl)-ethyl, or a benzyl radical substituted by nitro, chlorine, methyl or by methoxy.

11. N,O,O′,O″-tetra-(alkoxycarbonyl)-desferrioxamine B according to claim 1 with the exception of N,O,O′,O″-tetra-(ethoxycarbonyl)-desferrioxamine B.

12. N,O,O′, O″-tetra-[(3-oxa-, 3,6-dioxa- or 3,6,9-trioxa)-alkoxycarbonyl]-desferrioxamine B according to claim 1.

13. O,O′,O″-tri-(alkoxycarbonyl)-desferrioxamine B or a pharmaceutically acceptable acid addition salt thereof according to claim 1.

14. O,O′,O″-tri-[(3-oxa, 3,6-dioxa- or 3,6,9-trioxa)-alkoxycarbonyl]-desferrioxamine B or a pharmaceutically acceptable acid addition salt thereof according to claim 1.

15. O,O′,O″-tri-(3-oxa-butoxy-carbonyl)-desferrioxamine B or a pharmaceutically acceptable acid addition salt thereof according to claim 1.

16. O,O′,O″-tri-(3,6-dioxa-heptyloxycarbonyl)desferrioxamine B or a pharmaceutically acceptable acid addition salt thereof according to claim 1.

17. N-tert.-butoxycarbonyl-O,O′,O″-tri-(2-methoxyethoxy-carbonyl)-desferrioxamine B according to claim 1.

18. N-tert.-butoxycarbonyl-O,O′,O″-tri-[2-(2-methoxyethoxy)-ethoxy-carbonyl]-desferrioxamine B according to claim 1.

19. N-tert.-butoxycarbonyl-O,O′,O″-tri-octanoyl-desferrioxamine B according to claim 1.

20. A compound of the formula I according to any one of claims 1 to 19 or a pharmaceutically acceptable salt of such a compound having salt-forming properties for use in the therapeutic treatment of the human or animal body.

21. A compound according to any one of claims 1 to 19 for use in the formation of iron(III) and/or aluminium complexes in living cells.

22. Process for the manufacture of a compound of the formula I according to claim 1 or a salt thereof if that acylate has salt-forming properties, characterised in that

a) a compound of the formula

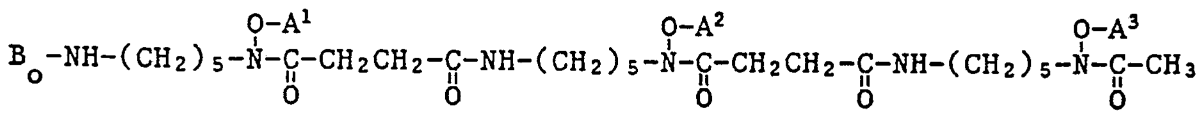

(II)

in which each of $A^1$, $A^2$ and $A^3$, independently of the others, is hydrogen or has one of the meanings of Ac defined in claim 1, and $B_o$ is hydrogen or, if at least one of the symbols $A^1$, $A^2$ and $A^3$ is hydrogen, may also be an amino-protecting group $X_o$ other than an oxycarbonyl group, is acylated using a reactive derivative of a carbonic acid monoester derived from the radical Cb defined in claim 1, or

b) a compound of the formula I in which B represents a radical Cb other than ethoxycarbonyl or benzyloxycarbonyl, and $AA^1$, $AA^2$ and $AA^3$ represent hydrogen is acylated to form a compound of the formula I in which B represents a radical Cb other than ethoxycarbonyl or benzyloxycarbonyl and each of $AA^1$, $AA^2$ and $AA^3$, independently of the others, has the meaning of Ac and/or Cb, or

c) a compound of the formula I in which at least one of the symbols $AA^1$, $AA^2$ and $AA^3$ has one of the meanings of Cb and B represents an amino-protecting group X is converted by removing this amino-protecting group, into a corresponding compound of the formula I in which B is hydrogen, or into a salt thereof, and/or,

if desired, a free compound of the formula I having salt-forming properties is converted into a salt and/or a compound of the formula I is freed from such a salt.

23. Process according to claim 22 [process a) or b)] characterised in that both the amino group and also free hydroxy groups are acylated with an excess of a chloroformic acid ester CbCl in which Cb has the meanings mentioned in claim 1, in the presence of a strongly basic acid-binding agent.

24. Process according to claim 22 [process a)], characterised in that the amino group is acylated selectively with an equivalent amount of a symmetrical carbonic acid anhydride of the formula Cb—O—Cb, in which Cb has the meanings given in claim 1, or of a corresponding active ester in the presence of a basic acid-binding agent.

25. Process according to any one of claims 22 to 26, characterised in that there is manufactured one of the compounds of the formula I characterised in claims 2 to 19.

26. Pharmaceutical compositions, containing as active ingredient a compound of the formula I according to any one of claims 1 to 19 or a pharmaceutically acceptable salt of such a compound having salt-forming properties together with a pharmaceutical carrier.

27. Pharmaceutical compositions according to claim 26 for oral administration.

28. Pharmaceutical compositions according to claim 27, which, owing to the pharmaceutical carrier, are not suitable for parenteral administration but are suitable for oral administration.

29. Use of a compound according to any one of claims 1 to 19 for the manufacture of a medicament for

the treatment of warm-blooded animals, including humans for pathological conditions associated with an excess of iron (III) or aluminium in the body.

30. Use of a compound according to any one of claims 1 to 19 for the manufacture of a medicament for the treatment of warm-blooded animals, including humans, for pathological conditions caused by iron(III)-dependent pathogenic organisms.

31. Use of a compound according to any one of claims 1 to 19 as an intermediate for the manufacture of therapeutically effective derivatives of desferrioxamine B.

**Claims for the Contracting State: AT**

1. Process for the manufacture of an acylate of desferrioxamine B of the formula

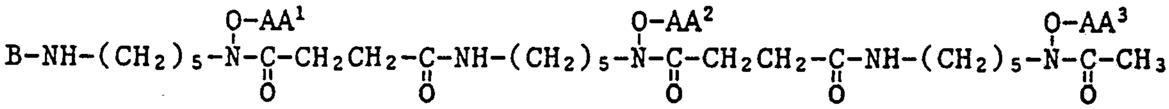

(I)

the symbols having the following meanings:

each of $AA^1$, $AA^2$ and $AA^3$, independently of the others, is an acyl radical referred to as Ac, of a carboxylic acid having from 1 to 24 carbon atoms, or an esterified oxycarbonyl radical referred to as Cb (acyl radical of a carbonic acid monoester) having a total of from 2 to 25 carbon atoms, and

B has one of the meanings of Cb other than ethoxycarbonyl or benzyloxycarbonyl, or, if at least one of the symbols $AA^1$, $AA^2$ and $AA^3$ represents Cb, it may also be hydrogen or an amino-protecting group selected from mono- or bicyclic α-aryl-lower alkoxycarbonyl radicals other than a benzyloxycarbonyl radical, from secondary or tertiary alkoxycarbonyl or cycloalkoxycarbonyl radicals, and from β-(trihydrocarbyl-silyl)-ethoxycarbonyl radicals and allyloxycarbonyl,

or a salt thereof if this acylate has salt-forming properties, characterised in that

a) a compound of the formula

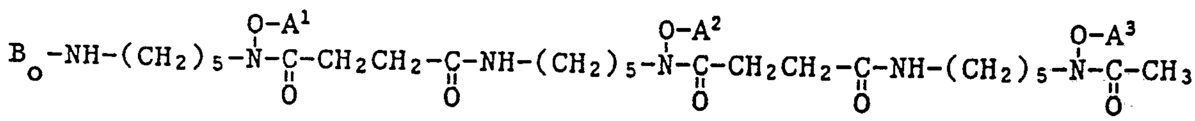

(II)

in which each of $A^1$, $A^2$ and $A^3$, independently of the others, is hydrogen or has one of the meanings of Ac defined in claim 1, and $B_o$ is hydrogen or, if at least one of the symbols $A^1$, $A^2$ and $A^3$ is hydrogen, may also be an amino-protecting group $X_o$ other than an oxycarbonyl group, is acylated using a reactive derivative of a carbonic acid monoester derived from the radical Cb defined in claim 1, or

b) a compound of the formula I in which B represents a radical Cb other than ethoxycarbonyl or benzyloxycarbonyl, and $AA^1$, $AA^2$ and $AA^3$ represent hydrogen is acylated to form a compound of the formula I in which B represents a radical Cb other than ethoxycarbonyl or benzyloxycarbonyl and each of $AA^1$, $AA^2$ and $AA^3$ independently of the others, has the meaning of Ac and/or Cb, or

c) a compound of the formula I in which at least one of the symbols $AA^1$, $AA^2$ and $AA^3$ has one of the meanings of Cb and B represents an amino-protecting group X is converted, by removing this amino-protecting group, into a corresponding compound of the formula I in which B is hydrogen, or into a salt thereof, and/or,

if desired, a free compound of the formula I having salt-forming properties is converted into a salt and/or a compound of the formula I is freed from such a salt.

2. Process according to claim 1 [process a) or b)], characterised in that both the amino group and also free hydroxy groups are acylated with an excess of a chloroformic acid ester CbCl in which Cb has the meanings mentioned in claim 1, in the presence of a strongly basic acid-binding agent.

3. Process according to claim 1 [process a)], characterised in that the amino group is acylated selectively with an equivalent amount of a symmetrical carbonic acid anhydride of the formula Cb—O—Cb— in which Cb has the meanings given in claim 1, or of a corresponding active ester in the presence of a basic acid-binding agent.

4. Process according to claim 1, characterised in that the starting materials are so chosen that there is manufactured a compound of the formula I in which the symbols have the following meanings: all of $AA^1$, $AA^2$ and $AA^3$ are acyl radicals, each of which, independently of the others, has one of the meanings of Ac, or all are oxycarbonyl radicals, each of which, independently of the others, has one of the meanings of Cb, or a salt of such a compound having salt-forming properties.

5. Process according to claim 1, characterised in that the starting materials are so chosen that there is manufactured a compound of the formula I in which $AA^1$, $AA^2$ and $AA^3$ all represent identical Ac or all

represent identical Cb, or a salt of such a compound having salt-forming properties.

6. Process according to claim 1, characterised in that the starting materials are so chosen that there is manufactured a compound of the formula I in which B represents hydrogen, one of the amino-protecting groups mentioned in claim 1 or an oxycarbonyl radical of the partial formula R—$CH_2$—O—CO— in which R is hydrogen or an unsubstituted or substituted hydrocarbyl radical having from 1 to 23 carbon atoms, other than methyl or a phenyl radical, and $AA^1$, $AA^2$ and $AA^3$, independently of one another, each represents an oxycarbonyl radical of the type defined above, or a pharmaceutically acceptable salt of such a compound having salt-forming properties.

7. Process according to claim 6, characterised in that the starting materials are so chosen that there is manufactured a compound of the formula I in which B represents hydrogen and $AA^1$, $AA^2$ and $AA^3$ each represents an alkoxycarbonyl radical whose carbon chain may be interrupted by 1, 2 or more non-vicinal oxygen atoms, or a ($C_6$—$C_{11}$-alkenyl)-oxycarbonyl radical, or a pharmaceutically acceptable acid addition salt thereof.

8. Process according to claim 6, characterised in that the starting materials are so chosen that there is manufactured a compound of the formula I in which B, $AA^1$, $AA^2$ and $AA^3$ each represents the same alkoxycarbonyl radical, other than ethoxycarbonyl, whose carbon chain may be interrupted by 1, 2 or more non-vicinal oxygen atoms, or a ($C_6$—$C_{11}$-alkenyl)-oxycarbonyl radical, or a pharmaceutically acceptable acid addition salt thereof.

9. Process according to claim 6, characterised in that the starting materials are so chosen that there is manufactured a compound of the formula I in which B represents one of the amino-protecting groups mentioned in claim 1 other than the oxycarbonyl radical of the formula $R_o$—O—CO—, in which $R_o$ is a hydrocarbyl radical that can be removed under neutral conditions and/or by acidolysis, and $AA^1$, $AA^2$ and $AA^3$ each represents an alkoxycarbonyl radical whose carbon chain may be interrupted by 1, 2 or more non-vicinal oxygen atoms, or a ($C_6$—$C_{11}$-alkenyl)-oxycarbonyl radical.

10. Process according to claim 1 or 6, characterised in that the starting materials are so chosen that there is manufactured a compound of the formula I in which B is an oxycarbonyl radical, other than ethoxycarbonyl or benzyloxycarbonyl, that can be used as an amino-protecting group and has the formula $R_o$—O—CO— in which $R_o$ is a hydrocarbyl radical that can be removed under neutral conditions and/or by acidolysis, and $AA^1$, $AA^2$ and $AA^3$ each represents an alkoxycarbonyl radical whose carbon chain may be interrupted by 1, 2 or more non-vicinal oxygen atoms, or a ($C_6$—$C_{11}$-alkenyl)-oxycarbonyl radical.

11. Process according to claim 1, characterised in that the starting materials are so chosen that there is manufactured a compound of the formula I in which B represents an oxycarbonyl radical, other than benzyloxycarbonyl, that can be used as an amino-protecting group and has the formula $R_o$—O—CO— in which $R_o$ is a hydrocarbyl radical that can be removed under neutral conditions and/or by acidolysis.

12. Process according to any one of claims 9 to 11, characterised in that the starting materials are so chosen that there is manufactured a compound of the formula I in which $R_o$ represents tert.-butyl, allyl, 2-(trimethylsilyl)-ethyl, or a benzyl radical substituted by nitro, chlorine, methyl or by methoxy.

13. Process according to claim 1, characterised in that the starting materials are so chosen that there is manufactured an N,O,O′,O″-tetra-(ethoxycarbonyl)-desferrioxamine B with the exception of N,O,O′,O″-tetra-(ethoxycarbonyl)-desferrioxamine B.

14. Process according to claim 1, characterised in that the starting materials are so chosen that there is manufactured an N-O-O′,O″-tetra-[(3-oxa-, 3,6-dioxa- or 3,6,9-trioxa)-alkoxycarbonyl]-desferrioxamine B.

15. Process according to claim 1, characterised in that the starting materials are so chosen that there is manufactured an O,O′,O″-tri-(alkoxycarbonyl)-desferrioxamine B or a pharmaceutically acceptable acid addition salt thereof.

16. Process according to claim 1, characterised in that the starting materials are so chosen that there is manufactured an O,O′,O″-tri-[(3-oxa-, 3,6-dioxa- or 3,6,9-trioxa)-alkoxycarbonyl]-desferrioxamine B or a pharmaceutically acceptable acid addition salt thereof.

17. Process according to claim 1, characterised in that the starting materials are so chosen that there is manufactured O,O′,O″-tri-(3-oxa-butoxy-carbonyl)-desferrioxamine B or a pharmaceutically acceptable acid addition salt thereof.

18. Process according to claim 1, characterised in that the starting materials are so chosen that there is manufactured O,O′,O″-tri-(3-6-dioxa-heptyloxycarbonyl)-desferrioxamine B or a pharmaceutically acceptable acid addition salt thereof.

19. Process according to claim 1, characterised in that the starting materials are so chosen that there is manufactured N-tert.-butoxycarbonyl-O,O′,O″-tri-(2-methoxy-ethoxy-carbonyl)-desferrioxamine B.

20. Process according to claim 1, characterised in that the starting materials are so chosen that there is manufactured N-tert.-butoxycarbonyl-O,O′,O″-tri-[2-(2-methoxy-ethoxy)-ethoxy-carbonyl]-desferrioxamine B.

21. Process according to claim 1, characterised in that the starting materials are so chosen that there is manufactured N-tert.-butoxycarbonyl-O,O′,O″-tri-octanoyldesferrioxamine B.